(19) Europäisches Patentamt
European Patent Office
Office européen des brevets

(11) **EP 3 391 046 B1**

(12) **FASCICULE DE BREVET EUROPEEN**

(45) Date de publication et mention de la délivrance du brevet:
**26.03.2025 Bulletin 2025/13**

(21) Numéro de dépôt: **16809887.9**

(22) Date de dépôt: **16.12.2016**

(51) Classification Internationale des Brevets (IPC):
**G01N 33/50** (2006.01)    **G01N 33/68** (2006.01)
**C12Q 1/68** (2018.01)    **C12Q 1/6883** (2018.01)

(52) Classification Coopérative des Brevets (CPC):
**G01N 33/5088; C12Q 1/6883; G01N 33/6881;**
C12Q 2600/148; C12Q 2600/158

(86) Numéro de dépôt international:
**PCT/EP2016/081562**

(87) Numéro de publication internationale:
**WO 2017/103195 (22.06.2017 Gazette 2017/25)**

(54) **PROCEDES D'EVALUATION DES EFFETS DE LA DESHYDRATATION SUR LA PEAU D'ENFANT**

VERFAHREN ZUR BEWERTUNG DER AUSWIRKUNGEN VON DEHYDRIERUNG AUF DIE HAUT VON KINDERN

METHOD FOR EVALUATING THE EFFECTS OF DEHYDRATION ON CHILDREN'S SKIN

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.12.2015 FR 1562528**

(43) Date de publication de la demande:
**24.10.2018 Bulletin 2018/43**

(73) Titulaire: **Laboratoires Expanscience**
**92048 Paris La Défense Cedex (FR)**

(72) Inventeurs:
• **BREDIF, Stéphanie**
**28210 Croisilles (FR)**
• **BAUDOUIN, Caroline**
**78120 Rambouillet (FR)**

(74) Mandataire: **Regimbeau**
**20, rue de Chazelles**
**75847 Paris Cedex 17 (FR)**

(56) Documents cités:
WO-A1-2014/009566    WO-A1-2015/104413
WO-A2-2014/170495    FR-A1- 2 912 916
FR-A1- 2 945 444    FR-A1- 2 983 868
FR-A1- 3 011 008    FR-A1- 3 019 186

• MARIKO YOKOTA ET AL: "The possible involvement of skin dryness on alterations of the dermal matrix", EXPERIMENTAL DERMATOLOGY, vol. 23, 19 September 2014 (2014-09-19), COPENHAGEN; DK, pages 27 - 31, XP055705154, ISSN: 0906-6705, DOI: 10.1111/exd.12392
• S LECLERE-BIENFAIT ET AL: "Avocado perseose, a biomimetic active ingredient for the protection and accompaniment of infants' skin", JOURNAL OF INVESTIGATIVE DERMATOLOGY, 1 May 2013 (2013-05-01), pages S106, XP055095186, Retrieved from the Internet <URL:http://www.nature.com/jid/journal/v133/n1s/pdf/jid201399a.pdf> [retrieved on 20140108], DOI: http://www.nature.com/jid/journal/v133/n1s/index.html#ab

**Description**

La peau est un ensemble de cellules et de macromolécules regroupées sous forme d'un tissu résistant et souple, recouvrant la totalité du corps. Elle est formée de deux couches jointes : l'épiderme et le derme auxquelles on peut associer les tissus sous-cutanés.

L'épiderme dont le principal rôle est la protection du corps constitue la couche la plus superficielle de la peau et assure l'imperméabilité de la peau et sa résistance. On peut identifier dans celui-ci quatre couches cellulaires distinctes, une couche basale (*stratum basalis*), une couche épineuse (*stratum spinosum*), une couche granuleuse (*stratum granulosum*), et une couche cornée (*stratum corneum*). Si différents types cellulaires coexistent dans l'épiderme, les kératinocytes sont largement majoritaires (90 %). Leur activité caractéristique est la synthèse des kératines, des protéines fibreuses et insolubles dans l'eau qui représentent 95 % des protéines totales de l'épiderme.

La peau a pour fonction principale d'établir une barrière de protection contre les atteintes de l'environnement tout en permettant certains échanges entre le milieu intérieur et le milieu extérieur. La fonction de barrière est assurée avant tout par la couche cornée (*stratum corneum*), laquelle rend la peau imperméable et hydrophobe, protégeant ainsi le derme d'une entrée massive d'eau. C'est également la couche cornée qui résiste aux agressions chimiques. Elle se compose de cellules, appelées cornéocytes, mortes et sans noyau, mais remplies de kératines et d'autres produits comme les lipides, les acides gras et les céramides. Les cornéocytes sont reliés par des jonctions serrées particulières, les cornéodesmosomes, formant une couche compacte dont la cohésion est encore renforcée par un ciment lipidique. Sous la couche granuleuse, les jonctions serrées dans la couche granuleuse participent aussi de la fonction de barrière de la peau (voir par exemple, Hogan et al., J Allergy, 2012 : 901940, 2012).

Au quotidien, la peau doit faire face à des attaques diverses. Elle est exposée, par exemple, à des agents chimiques comme le savon ainsi qu'à des stress physiques comme la friction avec les vêtements et l'exposition au soleil. L'épiderme et les annexes épidermiques doivent donc se renouveler constamment pour garder la peau en bon état. Ce sont les cellules souches qui rendent ces processus de maintien et de réparation possibles. Plus particulièrement, la capacité régénérative de l'épiderme est conférée par des cellules souches adultes qui permettent le remplacement régulier des cellules différenciées éliminées lors de la kératinisation. Les cellules souches épidermiques donnent ainsi naissance à des kératinocytes, qui se différencieront pour donner finalement des cornéocytes, lesquels sont exfoliés durant le processus de desquamation. Ce processus est en particulier crucial pour la maturation et le maintien de la fonction barrière.

La fonction barrière est particulièrement importante en particulier pour limiter les pertes d'eau de l'épiderme.

En effet, quel que soit le type de peau, celle-ci peut subir un état de déshydratation passager qui peut être lié à des facteurs extrinsèques (vent, froid, soleil, détergents...) et/ou intrinsèques (psoriasis, eczéma, sénescence...). Ce manque d'eau entraine une diminution de l'efficacité de la barrière cutanée. Une cascade d'évènements, appelée « cycle de la peau sèche », se met alors en place. Tout d'abord, la diminution de la fonction barrière entraine une amplification de la chute du contenu en eau ainsi que du défaut de barrière. En retour, la prolifération des kératinocytes augmente (hyperkératose) et des changements inflammatoires modérés sont induits, la peau essayant de se réparer elle-même. Cet état hyperprolifératif inflammatoire est crucial dans le cycle de la peau sèche car il conduit à une différenciation aberrante et à la production de matériaux et structures de mauvaise qualité. Enfin, l'activité réduite des enzymes de la desquamation entraine un épaississement et une perte d'hygroscopie de la couche cornée.

L'adaptation à la vie extra-utérine est un processus qui commence dès la naissance et se poursuit tout au long de la première année de la vie. Les premiers mois de la vie postnatale constituent une période de réorganisation structurelle et fonctionnelle de la peau qui permet une adaptation physiologique à l'environnement extra-utérin. Par exemple, l'immaturité de la peau du nouveau-né est soulignée par la différence de structure et de composition moléculaire du *stratum corneum* par rapport à l'adulte. Celles-ci sont incomplètes et continuent ainsi de se développer durant les 12 premiers mois au moins après la naissance (Chiou et al., Skin Pharmacol Physiol, 17: 57-66, 2004; Nikolovski et al., J Invest Dermatol, 128: 1728-1736, 2008; Stamatas et al., Pediatr Dermatol, 27: 125-131, 2010 ; Telofski et al., Dermatol Res Pract, 2012 : 198789, 2012). Par ailleurs, les résultats de deux études cliniques récentes (Fluhr et al., Br J Dermatol, 166(3) : 483-90, 2012 et Fluhr et al., Br J Dermatol, 2014, doi: 10.1111/bjd.12880) suggèrent que la peau des nourrissons présente une certaine immaturité sur sa capacité à capter l'eau et à réguler les mécanismes qui y sont liés. De plus ces travaux ont montré que la barrière épidermique s'organise structurellement de la naissance à l'âge de 2 ans et n'est donc pas complètement compétente pendant cette période. Ceci contribue à expliquer la fragilité de la peau des nourrissons et jeunes enfants et la susceptibilité de celle-ci aux agressions chimiques, physiques et microbiennes.

De plus, une maturation incomplète de la peau peut avoir des conséquences cliniques importantes. Il est donc important de permettre à la peau de se construire et de se développer correctement et harmonieusement, sans quoi son organisation fonctionnelle et structurelle pourrait en être obérée. À cet égard, il est crucial de préserver la fonction barrière et la capacité de renouvellement de l'épiderme.

Ainsi, l'immaturité de la barrière et des mécanismes de régulation de l'hydratation dans la peau du bébé pourrait rendre celle-ci encore plus vulnérable aux facteurs exogènes ou endogènes impliqués dans la sécheresse cutanée.

Il existe donc toujours un besoin, en intégrant les connaissances sur la peau de l'enfant et sur le rôle de la déshydratation pour identifier et caractériser des actifs et des formulations améliorés pour prendre en charge au mieux la peau sèche des enfants.

WO2014/009566 décrit un procédé d'identification de marqueurs moléculaires caractérisant la peau d'enfant, ainsi qu'un procédé d'évaluation de la tolérance et d'identification d'actifs ou formulations pour la peau d'enfant. WO2014/170495 concerne des méthodes d'identification d'actifs choisis parmi les sucres d'avocat en C7, pour prévenir ou traiter une déficience de la barrière cutanée de la peau d'enfant. Yokota et al., 2014 (Experimental Dermatology, vol. 23, 27-31) décrit des équivalents d'épiderme humain reconstruits (Episkin™ Large Model RHEE) cultivés dans des conditions de dessication induites par des ampoules contenant des granules de $CaCl_2$ placées sur les cultures.

DESCRIPTION

Les présents inventeurs ont montré que la peau des enfants, et en particulier celle des nourrissons, est particulièrement sensible à la déshydratation. Ils ont en particulier pu identifier des marqueurs biologiques dont l'expression est modifiée dans la peau sèche. De tels marqueurs sont particulièrement avantageux parce qu'ils permettent de suivre la réponse de la peau dans des conditions de dessiccation.

Les inventeurs ont développé des procédés d'évaluation de l'efficacité *in vitro* de principes actifs et de formulations sur la prévention des effets de la déshydratation pour la peau d'enfant, utilisant un modèle de peau spécifiquement capable de reproduire les caractéristiques de la peau des enfants, et en particulier celle des enfants très jeunes comme les nourrissons. Les études de l'art antérieur reposaient sur l'utilisation de la populations d'adultes pour analyser la réponse de la peau à la déshydratation (De Benedetto et al., J Allergy Clin Immunol., 127(3) : 773-786, 2011) ou sur des modèles de peau reconstruite d'adultes (Yokota et al., Exp Dermatol., 23 Suppl 1:27-31, 2014). Cependant, la propriétés de la peau évoluent durant les premières années de la vie (voir par exemple Fluhr et al., Exp Dermatol., 19(6) : 483-492, 2010 ; Fluhr et al., Br J Dermatol, 166(3) : 483-90, 2012 et Fluhr et al., Br J Dermatol., 2014, doi: 10.1111/bjd.12880) et il est peu probable qu'il soit possible de déterminer précisément les effets de la déshydratation sur la peau des enfants à partir d'échantillons de peau d'adulte.

En revanche, les inventeurs ont mis au point des modèles de peau reconstruite à partir de d'échantillons provenant d'enfant et ont pu tester l'effet de la déshydratation sur ces modèles. Ils ont ainsi pu observer que l'expression de certains marqueurs biologiques était altérée quand ces modèles de peau reconstruite d'enfant étaient cultivés dans des conditions de dessiccation. Certains marqueurs, comme les marqueurs de l'inflammation étaient ainsi plus fortement exprimés, tandis que l'expression d'autres, tels que les marqueurs des cellules souches ou ceux de la fonction barrière était diminuée. En revanche, les variations d'expression de ces marqueurs étaient réduites, voire estompées, quand les modèles étaient traités avec des actifs ou des formulations connues pour traiter ou prévenir la peau sèche. Ce résultat souligne la pertinence physiologique de ces marqueurs. L'importance de l'utilisation de modèles de peau reconstruite d'enfants et non pas d'adultes pour isoler de tels marqueurs en est d'ailleurs renforcée.

L'invention est notamment telle que définie dans les revendications.

L'invention a notamment pour objet un procédé d'évaluation de l'efficacité in vitro d'un actif ou d'une formulation sur la prévention des effets de la déshydratation sur la peau d'enfant, caractérisé en ce que ledit procédé comprend les étapes suivantes :

a) mettre en contact ledit actif ou ladite formulation avec un modèle de peau reconstruite, ledit modèle étant obtenu à partir d'un échantillon cutané provenant d'un enfant ;

b) cultiver le modèle de peau reconstruite de l'étape a) dans des conditions de dessiccation ;

c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souches ; et

d) évaluer l'efficacité dudit actif ou de ladite formulation en fonction du niveau de l'étape c).

L'invention a également pour objet un procédé d'évaluation de l'efficacité in vitro d'un actif ou d'une formulation dans la réduction des effets de la déshydratation sur la peau d'enfant, caractérisé en ce que ledit procédé comprend les étapes suivantes :

a) cultiver le modèle de peau reconstruite dans des conditions de dessiccation, ledit modèle étant obtenu à partir d'un échantillon cutané provenant d'un enfant ;

b) mettre en contact ledit actif ou ladite formulation avec le modèle de peau reconstruite de l'étape a) ;

c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et

d) évaluer l'efficacité dudit actif ou de ladite formulation en fonction du niveau de l'étape c).

L'invention a également pour objet un procédé d'identification d'un actif ou d'une formulation pour la prévention des effets de la déshydratation sur la peau d'enfant, caractérisé en ce que ledit procédé comprend les étapes suivantes :

a) mettre en contact un actif ou une formulation candidat avec un modèle de peau reconstruite, ledit modèle étant obtenu à partir d'un échantillon cutané provenant d'un enfant ;

b) cultiver le modèle de peau reconstruite de l'étape a) dans des conditions de dessiccation ;

c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et

d) déterminer si ledit actif ou ladite formulation candidat est une formulation ou un actif pour la prévention des effets de la déshydratation sur la peau d'enfant en fonction du niveau de l'étape c).

L'invention a également pour objet un procédé d'identification d'un actif ou d'une formulation pour la réduction des effets de la déshydratation sur la peau d'enfant, caractérisé en ce que ledit procédé comprend les étapes suivantes :

a) cultiver le modèle de peau reconstruite dans des conditions de dessiccation, ledit modèle étant obtenu à partir d'un échantillon cutané provenant d'un enfant ;

b) mettre en contact un actif ou une formulation candidat avec le modèle de peau de l'étape a) ;

c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et

d) déterminer si ledit actif ou ladite formulation candidat est une formulation ou un actif pour la réduction des effets de la déshydratation sur la peau d'enfant en fonction du niveau de l'étape c).

L'invention a également pour objet un procédé d'évaluation de la tolérance d'un actif ou d'une formulation sur la peau d'enfant déshydratée, ledit procédé comprenant les étapes suivantes :

a) mettre en contact ledit actif ou ladite formulation avec un modèle de peau reconstruite, ledit modèle étant obtenu à partir d'un échantillon cutané provenant d'un enfant ;

b) cultiver le modèle de peau reconstruite de l'étape a) dans des conditions de dessiccation en présence de l'actif ou de la formulation ;

c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et

d) déterminer si ledit agent actif, l'actif ou de la formulation est bien toléré par la peau d'enfant en fonction du niveau de l'étape c).

Par « peau sèche », on entend ici une peau qui a subi une perte d'eau. La peau sèche est une peau rêche, rugueuse, squameuse et inconfortable qui se caractérise par un déficit en eau de la couche cornée. La peau sèche est aussi connue comme étant une peau xérotique.

La peau sèche peut être causée par une exposition prolongée à faible humidité de sorte que le stress de déshydratation augmente dans une mesure telle que le gradient d'hydratation normale de la couche cornée est modifié. A cet égard, il a été montré qu'un taux d'humidité dans l'atmosphère inférieur à 30 % entraine une peau sèche (Sunwoo et al., J Physiol Anthropol, 25 : 7-14, 2006). Des conditions externes telles que, par exemple, un froid intense et sec, des irritations physiques ou chimiques, ou encore des expositions solaires, peuvent déclencher et/ou entretenir une déshydratation de la peau.

La peau sèche peut aussi se développer à la suite de changements physiques ou chimiques de la peau, tels que le vieillissement, entrainant la modification du gradient de l'hydratation normale. Ces changements peuvent notamment être occasionnés par des situations pathologiques, comme par exemple la dermatose atopique.

Par « condition de dessiccation », on entend ici toute condition conduisant à une sortie d'eau de la peau. Les conditions de dessiccation sont donc des conditions qui conduisent un modèle de peau d'enfant normalement hydratée à devenir sèche. La sortie d'eau dudit modèle peut être causée par des conditions extérieures dans lesquelles le taux d'humidité dans l'atmosphère est abaissé. Elle peut aussi être causée par une altération directe de la fonction barrière de la peau, par exemple par une altération des Natural Moisturizing Factors (NMF) ou du film lipidique de la couche cornée. Les inventeurs ont montré qu'un modèle de peau d'enfant cultivé dans des conditions dans lesquelles le taux d'humidité dans l'atmosphère est brutalement abaissé présente des caractéristiques physiologiques et histologiques tout à fait semblables à celles de la peau sèche d'enfant. Ces modèles de peau sèche mis au point par les inventeurs sont particulièrement avantageux, puisqu'ils permettent de reproduire le phénotype de la peau sèche d'enfant tel qu'observé *in vivo*. Ces modèles sont d'autant plus avantageux que d'autres modèles provoquant des sorties d'eau par altération de la couche lipidique ne permettent pas de mimer aussi bien les caractéristiques de la peau sèche d'enfant *in vivo.* Ainsi, à titre illustratif, les conditions de dessiccation sont des conditions dans lesquelles le taux d'humidité dans l'atmosphère est abaissé par rapport à des conditions usuelles de culture de cellules de peau. Par exemple, le taux d'humidité dans l'atmosphère est inférieur ou égal à 50 %. Par exemple, il est inférieur ou égal à 45 %, 40 %, 35 %, 30 % ou 25 %. Par exemple, il est inférieur ou égal à 25 %.

Par « enfant », on entend selon l'invention un individu dont l'âge est inférieur à 16 ans. Sont ainsi compris dans la catégorie des enfants, les nouveau-nés, dont l'âge est compris entre 0 et 1 mois, les nourrissons, qui ont entre 1 mois et 2 ans, et les enfants proprement dits, qui sont âgés d'au moins 2 ans. Un « nouveau-né », comme on l'entend ici, peut aussi bien être né à terme qu'être prématuré.

Pour lever toute ambiguïté, le terme « enfant » utilisé ici sans autre précision doit être entendu dans son acception la plus générale, c'est-à-dire comme se référant à une personne de moins de 16 ans. Un « adulte » est une personne qui n'est pas un enfant, autrement dit une personne âgée de plus de 16 ans.

Préférablement, la méthode peut être utilisée quelle que soit l'origine ethnique ou géographique de la peau, ou encore le phototype de celle-ci. Elle peut ainsi être d'origine caucasienne, africaine, asiatique, sud-américaine, mélanésienne ou autre ; elle peut encore présenter un phototype I, II, III, IV, V ou VI, sans que cela n'affecte le procédé. Celle-ci a en effet pour objet l'identification de marqueurs biologiques caractérisant n'importe quel type de peau et ne dépendant que de l'âge du donneur.

Les procédés de l'invention reposent donc sur l'utilisation d'un modèle de peau adapté, reproduisant la peau d'enfant, en particulier la peau sèche d'enfant, ainsi que sur l'utilisation de marqueurs biologiques, dont l'expression est affectée par la déshydratation de manière particulière dans la peau des enfants. L'invention permet ainsi de déterminer de façon précise quels actifs ont un effet avantageux sur la prévention ou le traitement des effets de la déshydratation de la peau. Les procédés de l'invention sont aussi adaptés à l'évaluation de l'activité de formulations. Les inventeurs ont ainsi pu montrer que certaines formulations étaient plus efficaces que d'autres pour prévenir et/ou limiter les effets de la peau sèche, démontrant ainsi l'utilité de l'approche entreprise.

Il est ici décrit une méthode d'évaluation de l'efficacité *in vitro* d'un actif ou d'une formulation pour prévenir ou traiter les effets de la déshydratation de la peau d'enfant, ladite méthode comprenant la détermination du niveau d'expression et/ou d'activation d'au moins un marqueur biologique.

Plus précisément, la méthode ici décrite comprend préférentiellement les étapes suivantes :

a) mettre en contact ledit actif ou ladite formulation avec un modèle de peau reconstruite, ledit modèle étant obtenu à partir d'un échantillon cutané provenant d'un enfant ;
b) cultiver le modèle de peau reconstruite de l'étape a) dans des conditions de dessiccation ;
c) mesurer le niveau d'expression d'au moins un marqueur biologique dans le modèle de peau de l'étape b) ; et
d) évaluer l'efficacité dudit actif ou de ladite formulation en fonction du niveau de l'étape c).

A titre illustratif, le modèle de peau reconstruite est cultivé dans des conditions de dessiccation dans l'étape b) en présence de l'actif ou de la formulation. Les inventeurs ont mis au point des modèles de peau sèche cultivés en atmosphère sèche permettant de reproduire le phénotype de la peau sèche d'enfant tel qu'observé *in vivo*. Ainsi, à titre d'exemple, les conditions de dessiccation sont des conditions de culture cellulaire dans lesquelles le taux d'humidité dans l'atmosphère est abaissé par rapport à des conditions usuelles de culture de cellules de peau. Par exemple, le taux d'humidité dans l'atmosphère est inférieur ou égal à 50 %. Par exemple, il est inférieur ou égal à 45 %, 40 %, 35 % ou 25 %. Selon un exemple particulier, il est inférieur ou égal à 25 %. Selon un autre exemple, l'actif ou la formulation est éliminée préalablement à l'exposition dudit modèle de peau reconstruite à la dessiccation lors de ladite étape b).

Par ailleurs, il est aussi ici décrit un procédé d'évaluation de l'efficacité *in vitro* d'un actif ou d'une formulation dans la réduction des effets de la déshydratation sur la peau d'enfant, caractérisé en ce que ledit procédé comprend les étapes suivantes:

a) cultiver dans des conditions de dessiccation un modèle de peau reconstruite, ledit modèle étant obtenu à partir d'un échantillon cutané provenant d'un enfant ;
b) mettre en contact ledit actif ou ladite formulation avec le modèle de peau reconstruite de l'étape a) ;
c) mesurer le niveau d'expression d'au moins un marqueur biologique dans le modèle de peau de l'étape b) ; et
d) évaluer l'efficacité dudit actif ou de ladite formulation en fonction du niveau de l'étape c).

L'homme du métier comprendra sans peine que les étapes a) et b) peuvent être réalisées simultanément ou successivement, selon ses besoins. Autrement dit, le modèle de peau reconstruite peut être cultivé dans des conditions de dessiccation dans l'étape b) en présence de l'actif ou de la formulation. Alternativement, le modèle de peau peut d'abord être cultivé dans des conditions de dessiccation, puis mis en contact avec l'actif ou la formulation.

Par « l'efficacité d'une formulation ou d'un actif dans la prévention ou la réduction des effets de la déshydratation de la peau d'enfant », on entend ici la capacité de la formulation ou de l'actif à annuler ou diminuer lesdits effets de la déshydratation de la peau d'enfant. La prévention s'entend dans le cas présent d'un traitement qui est administré avant le développement des effets de la déshydratation, tandis que la réduction correspond à un traitement qui est administré une fois que les effets de la déshydratation sont apparus.

A titre d'illustration, la formulation candidate ou l'actif candidat est efficace pour prévenir, traiter ou réduire les effets de la déshydratation de la peau d'enfant, si ladite formulation candidate ou ledit actif candidat permet d'augmenter ou maintenir les niveaux d'expression d'au moins un marqueur biologique de la fonction barrière, et/ou d'au moins un marqueur biologique préférentiellement exprimés dans les cellules souches, et/ou d'au moins un lipide, et/ou d'au moins un NMF. Alternativement ou en combinaison, la formulation candidate ou l'actif candidat est efficace pour prévenir, traiter ou réduire les effets de la déshydratation de la peau d'enfant, si ladite formulation candidate ou ledit actif candidat permet de diminuer ou maintenir les niveaux d'expression d'au moins un marqueur biologique de l'inflammation.

A titre illustratif, les conditions de dessiccation sont des conditions de culture cellulaire dans lesquelles le taux d'humidité dans l'atmosphère est abaissé par rapport à des conditions usuelles de culture de cellules de peau. Par exemple, le taux d'humidité dans l'atmosphère est inférieur ou égal à 50 %. Par exemple, il est inférieur ou égal à 45 %, 40 %, 35 %, 30 % ou 25 %. Par exemple, il est inférieur ou égal à 25 %. Selon un mode de réalisation plus préféré, le donneur de l'échantillon est plus particulièrement un donneur ayant un âge compris entre 0 et 1 mois, entre 1 mois et 2 ans ou entre 2 ans et 16 ans. Autrement dit, selon ce mode de réalisation, le donneur de l'échantillon est choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans. Plus préférentiellement, le donneur de l'échantillon est un nouveau-né ou un nourrisson.

A titre d'illustration, le niveau d'expression dudit marqueur biologique de l'étape c) est comparé à un niveau d'expression de référence.

Il est important de vérifier que les actifs et les formulations de l'invention sont bien tolérés. Par exemple, certains produits actuellement commercialisés pourraient entrainer des irritations s'ils sont employés régulièrement. Un tel effet ne peut qu'empirer un état de peau sèche en développement ou déjà présent.

Il est donc ici décrit un procédé d'évaluation de la tolérance d'un actif ou d'une formulation lorsque la peau d'enfant est asséchée, ledit procédé comprenant les étapes suivantes :

a) mettre en contact ledit actif ou ladite formulation avec un modèle de peau reconstruite, ledit modèle étant obtenu à partir d'un échantillon cutané provenant d'un enfant ;
b) cultiver dans des conditions de dessiccation le modèle de peau reconstruite de l'étape a) en présence de l'actif ou de la formulation ;
c) mesurer le niveau d'expression d'au moins un marqueur biologique dans le modèle de peau de l'étape b) ; et
d) déterminer si ledit agent actif, l'actif ou de la formulation est bien toléré par la peau d'enfant en fonction du niveau de l'étape c).

Le procédé peut par exemple en outre comporter une comparaison de la viabilité cellulaire dans le modèle de peau reconstruite traité avec l'actif, la composition ou la formulation et dans le modèle de peau reconstruite témoin, c'est-à-dire une peau déshydratée mais qui n'a pas été traitée par l'actif ou la formulation. Dans ce cas, l'actif ou la formulation cosmétique est bien toléré par la peau d'enfant si la viabilité cellulaire du modèle de peau reconstruite n'est pas affectée par la présence de l'agent ou de la formulation.

Selon un autre exemple, le procédé comprend donc une étape supplémentaire de détermination de la viabilité cellulaire dans le modèle de peau reconstruite déshydratée et traité avec l'actif ou la formulation cosmétique, de détermination de la viabilité cellulaire du modèle de peau reconstruite témoin et de comparaison entre les deux.

De nombreux tests pour déterminer la viabilité cellulaire sont disponibles pour l'homme du métier et sont couramment utilisés en cosmétologie. On citera en particulier le test MTT, décrit par exemple dans Mosman et al. (J Immunol Methods, 65(1-2) : 55-63, 1983).

A titre illustratif, la formulation candidate ou l'actif candidat est bien toléré par la peau d'enfant, si ladite formulation candidate ou ledit actif candidat permet d'augmenter ou maintenir les niveaux d'expression d'au moins un marqueur biologique de la fonction barrière, et/ou d'au moins un marqueur biologique préférentiellement exprimés dans les cellules souches, et/ou d'au moins un lipide, et/ou d'au moins un NMF. Alternativement ou en combinaison, la formulation candidate ou l'actif candidat est bien toléré par la peau d'enfant, si ladite formulation candidate ou ledit actif candidat permet de diminuer ou maintenir les niveaux d'expression d'au moins un marqueur biologique de l'inflammation.

Selon un autre exemple, les conditions de dessiccation sont des conditions de culture cellulaire dans lesquelles le taux d'humidité dans l'atmosphère est abaissé par rapport à des conditions usuelles de culture de cellules de peau. Par exemple, le taux d'humidité dans l'atmosphère est inférieur ou égal à 50 %. Par exemple, il est inférieur ou égal à 45 %, 40 %, 35 %, 30 % ou 25 %. Selon un exemple particulier, il est inférieur ou égal à 25 %.

Dans un autre exemple, le niveau d'expression dudit marqueur biologique de l'étape c) est comparé à un niveau d'expression de référence.

Dans un autre aspect, l'invention permet d'isoler des formulations ou des actifs ayant un effet dans la prévention des effets de la déshydratation de la peau d'enfant. Comme le montrent les exemples expérimentaux, l'invention permet en particulier de distinguer les actifs ou les formulations selon leur activité pour prévenir les effets de la déshydratation de la peau d'enfant. L'invention est donc particulièrement adaptée pour identifier des formulations ou des actifs appropriés à cette peau très spécifique.

Il est donc également ici décrit un procédé d'identification d'un actif ou d'une formulation pour la prévention des effets de la déshydratation de la peau d'enfant, caractérisé en ce que ledit procédé comprend les étapes suivantes :

a) mettre en contact un actif ou une formulation candidat avec un modèle de peau reconstruite, ledit modèle étant obtenu à partir d'un échantillon cutané provenant d'un enfant ;

b) cultiver dans des conditions de dessiccation le modèle de peau de l'étape a) ;

c) mesurer le niveau d'expression d'au moins un marqueur biologique dans le modèle de peau de l'étape b) ; et

d) déterminer si ledit actif ou ladite formulation candidat est une formulation ou un actif pour la prévention des effets de la déshydratation de la peau d'enfant en fonction du niveau de l'étape c).

A titre illustratif, le modèle de peau reconstruite est cultivé en conditions de dessiccation dans l'étape b) en présence de l'actif ou de la formulation. Selon un autre exemple, l'actif ou la formulation est éliminée préalablement à l'exposition dudit modèle de peau reconstruite à la dessiccation lors de ladite étape b). Selon un autre exemple, les conditions de dessiccation sont des conditions de culture cellulaire dans lesquelles le taux d'humidité dans l'atmosphère est abaissé par rapport à des conditions usuelles de culture de cellules de peau. Par exemple, le taux d'humidité dans l'atmosphère est inférieur ou égal à 50 %. Par exemple, il est inférieur ou égal à 45 %, 40 %, 35 %, 30 % ou 25 %. Selon un exemple particulier, il est inférieur ou égal à 25 %.

De même, la méthode de l'invention permet d'isoler des actifs ou des formulations permettant de réduire les effets de la déshydratation de la peau d'enfant. Il est donc ici décrit cette méthode, comprenant les étapes suivantes :

a) cultiver dans des conditions de dessiccation un modèle de peau reconstruite, ledit modèle étant obtenu à partir d'un échantillon cutané provenant d'un enfant ;

b) mettre en contact un actif ou une formulation candidat avec le modèle de peau de l'étape a) ;

c) mesurer le niveau d'expression d'au moins un marqueur biologique dans le modèle de peau de l'étape b) ; et

d) déterminer si ledit actif ou ladite formulation candidat est une formulation ou un actif pour la réduction des effets de la déshydratation de la peau d'enfant en fonction du niveau de l'étape c).

L'homme du métier comprendra sans peine que les étapes a) et b) peuvent être réalisées simultanément ou successivement, selon ses besoins. Autrement dit, le modèle de peau reconstruite peut être cultivé en conditions de dessiccation dans l'étape b) en présence de l'actif ou de la formulation. Alternativement, le modèle de peau peut d'abord être cultivé en conditions de dessiccation, puis mis en contact avec l'actif ou la formulation.A titre illustratif, les conditions de dessiccation sont des conditions de culture cellulaire dans lesquelles le taux d'humidité dans l'atmosphère est abaissé par rapport à des conditions usuelles de culture de cellules de peau. Par exemple, le taux d'humidité dans l'atmosphère est inférieur ou égal à 50 %. Par exemple, il est inférieur ou égal à 45 %, 40 %, 35 %, 30 % ou 25 %. Selon un exemple particulier, il est inférieur ou égal à 25 %.

Dans un autre exemple, le niveau d'expression dudit marqueur biologique de l'étape c) est comparé à un niveau d'expression de référence.

La formulation candidate est une formulation pour la prévention ou la réduction des effets de la déshydratation de la peau d'enfant, si ladite formulation candidate permet de moduler l'expression d'au moins un marqueur biologique de l'invention. Cette modulation peut correspondre, selon les cas, et en particulier selon la nature du marqueur biologique, à une augmentation ou à une diminution de l'expression dudit marqueur. Par exemple, il peut être intéressant d'isoler des formulations minimisant les effets de la déshydratation sur les marqueurs préférentiellement exprimés dans les cellules souches, ces formulations permettant de préserver la capacité de renouvellement de l'épiderme fragile des enfants. De même, il serait avantageux d'identifier des formulations minimisant les effets de la déshydratation sur les marqueurs de la barrière chez les enfants, afin de maintenir l'intégrité de la barrière cutanée. Enfin, il pourrait être désirable d'isoler des formulations qui n'induiraient pas les marqueurs de l'inflammation. Ainsi, a titre illustratif, la formulation candidate est une formulation pour la prévention ou la réduction des effets de la déshydratation de la peau d'enfant, si ladite formulation candidate permet d'augmenter ou maintenir les niveaux d'expression d'au moins un marqueur biologique de la fonction barrière, et/ou d'au moins un marqueur biologique préférentiellement exprimés dans les cellules souches, et/ou d'au moins un lipide, et/ou d'au moins un NMF. Alternativement ou en combinaison, la formulation candidate est une formulation pour la prévention ou la réduction des effets de la déshydratation de la peau d'enfant, si ladite formulation candidate permet de diminuer ou maintenir les niveaux d'expression d'au moins un marqueur biologique de l'inflammation.

De la même façon, l'actif candidat est un actif pour la prévention ou la réduction des effets de la déshydratation sur la peau d'enfant, si ledit actif candidat permet de moduler l'expression d'au moins un marqueur biologique. Cette modulation peut correspondre, selon les cas, et en particulier selon la nature du marqueur biologique, à une augmentation ou à une diminution de l'expression dudit marqueur. Selon un mode de réalisation préféré, l'actif candidat est un actif pour la prévention ou la réduction des effets de la déshydratation de la peau d'enfant, si ledit actif candidat permet d'augmenter ou maintenir les niveaux d'expression d'au moins un marqueur biologique de la fonction barrière, et/ou d'au moins un marqueur biologique préférentiellement exprimés dans les cellules souches, et/ou d'au moins un lipide, et/ou d'au moins un NMF. Alternativement ou en combinaison, l'actif candidat est un actif pour la prévention ou la réduction des effets de la déshydratation de la peau d'enfant, si ledit actif candidat permet de diminuer ou maintenir les niveaux d'expression d'au moins un marqueur biologique de l'inflammation.

Dans un premier temps, la formulation ou l'actif d'intérêt est mis en contact avec une culture de peau reconstruite obtenue à partir d'un échantillon provenant d'un enfant. Cette mise en contact de l'actif d'intérêt avec le modèle de peau peut être faite directement. Alternativement, il pourra être avantageux de formuler l'actif d'intérêt, par exemple de façon à obtenir une composition liquide, afin de faciliter sa mise en contact avec le modèle de peau. Ainsi, à titre illustratif, le procédé comprend en outre une étape de formulation de l'actif, notamment sous forme d'une solution liquide, en particulier aqueuse, préalablement à l'étape de mise en contact dudit actif avec un modèle de peau.

Les inventeurs ont précédemment montré que les profils d'expression de catégories spécifiques de gènes (par exemple, gènes de la barrière, de l'inflammation, de défense, des cellules souches) évolue en fonction de l'âge (demande WO 2014/009566). L'homme du métier peut ainsi aisément caractériser la peau au niveau moléculaire depuis la naissance jusqu'à l'âge adulte. Plus particulièrement l'homme du métier notera que les cellules de la peau d'enfant présentent un profil d'expression spécifique des gènes impliqués dans des processus physiologiques particuliers, notamment le métabolisme cellulaire, la réponse au stress, l'inflammation, l'immunité, l'apoptose, la croissance/prolifération et le cycle cellulaire, la signalisation cellulaire, la migration et la différenciation, la barrière épidermique, l'adhésion et les cellules souches pluripotentes de la peau.

Le modèle de peau reconstruite obtenu à partir d'un échantillon cutané provenant d'un enfant peut être tout modèle tissulaire comprenant des cellules de la peau, notamment des kératinocytes, et dans lequel lesdites cellules cutanées ont été obtenues à partir d'un échantillon provenant d'un enfant.

Par « échantillon cutané », on entend ici tout échantillon contenant des cellules de la peau. Les échantillons cutanés comprennent donc aussi bien les explants de peau frais obtenus directement du patient, que les cultures de cellules cutanées en suspension, les cultures de cellules cutanées en monocouche, les cultures de cellules cutanées en bicouche et les modèles tissulaires, dont les cultures de peaux reconstruites et les cultures de muqueuses reconstruites. Comme il est souvent difficile de travailler sur des explants frais, il est particulièrement avantageux d'utiliser des cultures de cellules cutanées. Avantageusement, les cellules cutanées comprennent des cellules normales, saines ou pathologiques, ou des cellules issues de lignées. Par exemple, les cellules cutanées mises en culture peuvent être des cellules obtenues à partir d'explant de tissu cutané. Par « explant » ou « explant de peau », on entend ici un prélèvement de cellules ou de tissu cutané, lequel peut être réalisé dans un but chirurgical ou pour effectuer des analyses.

En particulier, un explant peut être obtenu lors d'une exérèse chirurgicale. Par « exérèse », on entend ici une intervention chirurgicale consistant à découper (exciser) une partie plus ou moins large ou profonde de la peau pour en traiter une anomalie ou une excroissance. On procède à une exérèse soit pour retirer une tumeur cancéreuse ou suspecte de l'être, soit pour traiter une anomalie bénigne de la peau qui est gênante, que ce soit pour des raisons fonctionnelles ou esthétiques. Une exérèse inclut par exemple les échantillons de peau obtenus après chirurgie plastique (plastie mammaire, abdominale, lifting, prélèvement préputial, otoplastie, c'est-à-dire recollement d'oreille, syndactylie ou doigt surnuméraire, etc.).

Un explant peut aussi être obtenu par biopsie. Par « biopsie », on entend ici un prélèvement de cellules ou tissu cutané réalisé à des fins d'analyse. Plusieurs types de procédures de biopsies sont connus et pratiqués dans le domaine. Les types les plus communs comprennent (1) la biopsie incisionnelle, dans laquelle seul un échantillon du tissu est prélevé ; (2) la biopsie excisionnelle (ou biopsie chirurgicale) qui consiste en l'ablation totale d'une masse tumorale, réalisant ainsi un geste thérapeutique et diagnostique, et (3) la biopsie à l'aiguille, dans laquelle un échantillon de tissu est prélevé avec une aiguille, celle-ci pouvant être large ou fine. D'autres types de biopsie existent, comme par exemple le frottis ou le curettage, et sont aussi englobés ici.

Alternativement, lesdites cellules cutanées peuvent être obtenues par différentiation de cellules souches (Guenou et al., Lancet, 374(9703) : 1745-1753, 2009 ; Nissan et al., Proc. Natl. Acad. Sci., 108(36) : 14861-14866, 2011 ; Kraehenbuehl et al., Nature Methods, 8 : 731-736, 2011).

Les cellules cutanées, qu'elles proviennent d'une biopsie ou qu'elles soient obtenues par différentiation de cellules souches, comprennent au moins un type de cellules habituellement présentes dans l'hypoderme, le derme et/ou l'épiderme. Ces cellules comprennent ainsi, entre autres, des kératinocytes, des mélanocytes, des fibroblastes, des adipocytes, des cellules endothéliales, des mastocytes, des cellules de Langerhans et/ou des cellules de Merkel. De façon préférentielle, les cellules cutanées selon l'invention comprennent au moins des kératinocytes ou des fibroblastes. A titre illustratif, les cellules cutanées comprennent des kératinocytes et/ou des fibroblastes.

De nombreuses méthodes de culture de cellules cutanées sont connues de l'homme du métier. N'importe laquelle de ces méthodes peut être utilisée pour cultiver les cellules cutanées de l'invention. Avantageusement, les cellules cutanées sont cultivées et/ou conservées dans des conditions maintenant, au moins partiellement, un métabolisme cellulaire et/ou des fonctions cellulaires. La culture de cellules cutanées ici décrite comprend donc aussi bien les cultures de cellules cutanées en suspension, les cultures de cellules cutanées en monocouche ou les cultures de cellules cutanées en bicouche que des modèles tissulaires, dont les cultures de peaux reconstruites et les cultures de muqueuses reconstruites.

Par exemple, les cultures de cellules cutanées en suspension sont pratiquées en routine dans un très grand nombre de laboratoires et ce, depuis plusieurs dizaines d'années. De même, les cultures de cellules cutanées en monocouche ou en bicouche sont connues et utilisées depuis très longtemps.

Par ailleurs, de nombreux modèles tissulaires, dont en particulier des modèles de peaux reconstruites et des modèles de muqueuses reconstruites (Rosdy et al., In Vitro Toxicol., 10(1) : 39-47, 1997 ; Ponec et al., J Invest Dermatol., 109(3) : 348-355, 1997 ; Ponec et al., Int J Pharm., 203(1-2) : 211-225, 2000; Schmalz et al., Eur J Oral Sci., 108(5) : 442-448, 2000 ; Black et al., Tissue Eng, 11(5-6) : 723-733, 2005 ; Dongari-Batgtzoglou et Kashleva, Nat Protoc, 1(4) : 2012-2018, 2006 ; Bechtoille et al., Tissue Eng, 13(11) : 2667-2679, 2007 ; Vrana et al., Invest Ophthalmol Vis Sci, 49(12) : 5325-5331, 2008 ; Kinicoglu et al., Biomaterials, 30(32) : 6418-6425, 2009 ; Auxenfans et al., Eur J Dermatol, 19(2) : 107-113, 2009 ; Kinicoglu et al., Biomaterials, 32(25) : 5756-5764, 2011 ; Costin et al., Altern Lab Anim, 39(4) : 317-337, 2011 ; Auxenfans et al., J Tissue Eng Regen Med, 6(7) : 512-518, 2012 ; Lequeux et al., Skin Pharmacol Physiol, 25(1) : 47-55, 2012; EP 29 678 ; EP 285 471 ; EP 789 074 ; EP 1 451 302 B1 ; EP 1 878 790 B1 ; EP 1 974 718 ; US 2007/0148,771 ; US 2010/0,099,576 ; WO 02/070729 ; WO 2006/063864 ; WO 2006/0,63865 ; WO 2007/064305) sont à la disposition de l'homme du métier.

Avantageusement, le modèle tissulaire comprend les modèles de peau reconstruite et les modèles de muqueuse reconstruite. A titre illustratif, le modèle de peau reconstruite est sélectionné dans le groupe comprenant les modèles de derme, contenant principalement des cellules stromales, et plus particulièrement encore des fibroblastes, les modèles d'épiderme constitué principalement de kératinocytes, les modèles d'hypoderme, les modèles de peau comprenant un derme et un épiderme, et les modèles de peau comprenant un derme, un épiderme et un hypoderme. Les modèles comprenant au moins un derme, forment des tissus de type conjonctifs, tandis que les modèles comprenant au moins un épiderme forment des épithélia stratifiés comprenant les couches caractéristiques du tissu considéré. Par exemple, on peut identifier dans les modèles d'épiderme une couche basale (*stratum basalis*), une couche épineuse (*stratum spinosum*), une couche granuleuse (*stratum granulosum*), et une couche cornée (*stratum corneum*). D'autre part, le modèle de muqueuse reconstruite est par exemple un modèle de muqueuse de la bouche, de la gencive, du vagin ou de la cornée.

Avantageusement, ledit modèle est un modèle tissulaire de type conjonctif de matrice de derme comprenant un support matriciel de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, une membrane ou un film d'acide polyglycolique.

Dans ce groupe on trouve par exemple les modèles dermiques Skin²™ modèle ZK1100 et Dermagraft® et Transcyte® (Advanced Tissue Sciences) ;

- un plastique traité culture cellulaire (formation d'un feuillet dermique : Michel et al., In Vitro Cell. Dev Biol. -Animal, 35 : 318-326, 1999) ;
- un gel ou une membrane à base d'acide hyaluronique (Hyalograft® 3D - Fidia Advanced Biopolymers) et/ou de collagène (comme par exemple un derme équivalent ou des lattices de collagène) et/ou de fibronectine et/ou de fibrine ; dans ce groupe on trouve par exemple le modèle dermique Vitrix® (Organogenesis) ;
- une matrice poreuse, surfacée ou non surfacée (par exemple un derme équivalent), réalisée à partir de collagène pouvant contenir un ou plusieurs glycosaminoglycanes et/ou éventuellement du chitosane (EP0296078A1, WO 01/911821 et WO 01/92322).

Dans ce groupe on trouve par exemple le modèle dermique Mimederm® (Coletica).
Ces supports matriciels comprennent des cellules stromales, en particulier des fibroblastes.
Avantageusement, ledit modèle de peau est un modèle d'épiderme comprenant un support matriciel de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable ;

dans ce groupe on trouve les modèles Epiderme reconstruits (Skinethic®) ainsi que le modèle EpiDerm®, (Mattek Corporation) ;

- un film ou une membrane à base d'acide hyaluronique et/ou de collagène et/ou de fibronectine et/ou de fibrine.

Dans ce groupe on peut citer particulièrement les modèles : Laserskin® (Fidia Advanced Biopolymers), Episkin ® (L'Oréal).
Ces modèles peuvent être ensemencés par des fibroblastes dans la partie dermique.
Ces modèles, dans lesquels peuvent être intégrés ou non des fibroblastes, servent de support pour l'ensemencement des kératinocytes et la reconstitution de l'épiderme. Avantageusement, sont introduites, outre les kératinocytes, des cellules pigmentaires, des cellules immunocompétentes, des cellules nerveuses ; de préférence les cellules immunocompétentes sont des cellules de Langerhans.
Avantageusement, ledit modèle tissulaire est un modèle tissulaire de peau ou de muqueuse reconstruite comprenant un support matriciel dermique ou de chorion de préférence choisi parmi :

- un support inerte choisi parmi le groupe consistant d'une membrane synthétique semi-perméable, en particulier une membrane de nitrocellulose semi-perméable, une membrane de nylon semi- perméable, une membrane ou une éponge de téflon, une membrane de polycarbonate ou de polyéthylène, polypropylène, de polyéthylène téréphtalate (PET) semi-perméable, une membrane inorganique Anopore semi-perméable, d'acétate ou d'ester de cellulose (HATF), une membrane Biopore-CM semi-perméable, une membrane de polyester semi-perméable, ledit support inerte contenant ou non des cellules stromales, en particulier des fibroblastes,
- un gel à base de collagène et/ou acide hyaluronique et/ou fibronectine, et/ou de fibrine comprenant des cellules stromales en particulier des fibroblastes,
- une matrice poreuse, surfacée ou non surfacée, réalisée à partir de collagène pouvant contenir un ou plusieurs glycosaminoglycanes et/ou éventuellement du chitosane, ces matrices poreuses intégrant des cellules stromales, en particulier des fibroblastes,
- un derme desépidermisé ou derme mort, humain ou animal.

Dans ce groupe, on peut citer particulièrement les modèles Mimeskin (Coletica), EpidermFT™, EpiAirway™, EpiOccular™ EpiOral™, EpiGingival™, EpiVaginal™ (MatTek corporation), Human Corneal Epithelium (HCE), Human Oral Epithelium (HOE), Human Gingival Epithelium (HGE), Human Vaginal Epithelium (HVE) (Skinethic®), Phenion® Full Thickness Skin Model (Phenion) Apligraf® (Organogenesis), ATS-2000 (CellSystems® Biotechnologie Vertrieb) ainsi que Skin 2TM (ZK1200-1300-2000 Advanced Tissue Science).

Il existe par ailleurs des modèles dédiés à la thérapie tissulaire qui peuvent également être utilisés dans le cadre de la présente invention. On peut citer les modèles Epidex (Modex Thérapeutiques), Epibase® (Laboratoire Genévrier), Epicell™ (Genzyme), Autoderm™ et Transderm™ (Innogenetics).

Le support matriciel est ensuite ensemencé par des kératinocytes pour reconstruire l'épiderme et obtenir finalement une peau reconstruite.

Avantageusement, le modèle de peau utilisé comprend un modèle dans lequel a été incorporé au moins un type cellulaire complémentaire, comme des cellules endothéliales (CE) et/ou des cellules immunitaires telles que lymphocytes, macrophages, mastocytes, cellules dendritiques et/ou des cellules adipeuses et/ou des annexes cutanées, comme des poils, des cheveux, des glandes sébacées.

Après avoir exposé le modèle de peau reconstruite à des conditions de dessiccation, l'homme du métier pourra procéder à la mesure du niveau d'expression des marqueurs biologiques.

Par « marqueur biologique », on entend ici une caractéristique qui est objectivement mesurée et évaluée comme indicateur de processus biologiques normaux, de processus pathogéniques, ou de réponses pharmacologiques à une intervention thérapeutique. Un marqueur biologique désigne donc toute une gamme de substances et de paramètre divers. Par exemple, un marqueur biologique peut être une substance dont la détection indique un état pathologique particulier (par exemple la présence de la protéine C activée en tant que marqueur d'une infection), ou au contraire une substance dont la détection indique un état physiologique spécifique. Le marqueur biologique est par exemple un gène, les produits d'un gène tels que ses transcrits et les peptides issus de ses transcrits, un lipide, un sucre ou un métabolite.

A titre illustratif, le marqueur biologique est un gène, les produits d'un gène tels que des transcrits ou des peptides, un lipide, un sucre ou un métabolite dont les changements d'expression, en particulier de niveau d'expression, corrèlent avec un état physiologique de la peau d'enfant.

L'homme du métier cherchant à déterminer à quelle classe appartient un marqueur génique ou protéique pourra facilement consulter la littérature scientifique pertinente ou se rapporter à des bases de données publiques telles que, par exemple, celles regroupées sur le site internet du National Center for Biotechnology Information (http://www.ncbi.nlm.nih.gov/guide/).

Les inventeurs ont particulièrement sélectionné des marqueurs dont la variation du niveau d'expression varie après déshydratation de manière surprenante et inattendue chez les enfants. Les marqueurs sélectionnés présentent donc un intérêt particulier dans le cadre de la méthode de l'invention, dans la mesure où leur niveau d'expression est mesuré sur un modèle de peau reproduisant les caractéristiques de la peau d'enfant.

Les inventeurs ont en particulier montré que les conditions de dessiccation affectaient la quantité de lipides et de NMF produits par un modèle de peau d'enfant. En outre, les inventeurs ont montré que dans le même temps, l'expression des marqueurs de la barrière diminue, ainsi que ceux de la synthèse du NMF. Enfin, les marqueurs des cellules souches sont eux-aussi affectés et par conséquent la capacité de renouvellement de l'épiderme. En revanche, la mise en contact de la peau avec une formulation active contre les effets de la déshydratation de la peau permet de prévenir et corriger les variations d'expression desdits marqueurs, ce qui souligne la pertinence de ceux-ci.

Le marqueur biologique est donc avantageusement un marqueur sélectionné dans le groupe des lipides du stratum corneum, notamment des céramides, des NMF, des marqueurs de l'inflammation, des marqueurs de la fonction barrière et des marqueurs préférentiellement exprimés dans les cellules souches.

Les inventeurs ont en particulier montré que la quantité de lipides de la peau diminuait quand le modèle de peau d'enfant était cultivé dans des conditions de dessiccation. Dans la couche cornée, les lipides sont disposés en plan lamellaires dans les espaces entre les cornéocytes, formant ainsi un ciment qui participe à la protection de la peau contre les agressions extérieures et au maintien d'un bon niveau d'eau intraépidermique. Ces lipides sont des phospholipides, cholestérol et glucosylcéramides, qui sont modifiés dans les espaces intercornéocytaires, par des enzymes spécialisées, en céramides, cholestérol, sulfate de cholestérol et acides gras libres. (Jungerstend et al., Contact Dermitis, 58(5) : 255-262, 2008). On retrouve également des céramides, des stérols et des acides gras libres dans le sébum, entre autres composants. Enfin, la surface de la peau est recouverte d'un film protecteur qui permet à la peau de maintenir son hydratation et de se protéger contre les agressions extérieures. Ce film cutané de surface comprend, entre autres, un film hydrolipidique, lequel est constitué essentiellement de sueur, d'eau, de sébum et autres lipides. Ceux-ci comprennent des céramides, des triglycérides et des acides gras, en proportions à peu près égales.

Selon un exemple particulier, le marqueur biologique est donc un marqueur lipidique. Par « lipides », on entend ici toute molécule naturelle liposoluble (c'est-à-dire lipophile). Les lipides sont un groupe hétérogène de composés possédant de nombreuses fonctions biologiques essentielles. Les lipides peuvent être définis plus particulièrement comme des petites molécules hydrophobes ou amphiphiles, qui proviennent entièrement ou en partie de groupes cétoacyl ou isoprènes. Pour un aperçu d'ensemble de toutes les classes de lipides, on se reportera avantageusement à « Lipid Metabolites and Pathways Strategy (LIPID MAPS) classification system » (National Institute of General Medical Sciences, Bethesda, MD). En particulier, le marqueur lipidique est choisi parmi les céramides, les phospholipides, les glucosylcéramides, les stérols, les triglycérides et les acides gras libres.A titre illustratif, le marqueur lipidique l'invention est choisi parmi les céramides.

Un « céramide » est un lipide issu de la réaction d'amidification de la sphingosine et d'un acide gras. Un céramide est donc constitué d'une base sphingosine ou phytoshingosine liée par une liaison amide à des acides $\alpha$-hydroxy, $\omega$-hydroxy ou non hydroxy possédant des longueurs de chaîne hydrophobe variables. Dans le *stratum corneum* humain, 9 classes de céramides, dénommés CER 1 à 9, ont été identifiées (voir par exemple Dayan, Cosm & Toil, 121(1) : 37-44, 2006 ; Jungerstend et al., Contact Dermitis, 58(5) : 255-262, 2008 ; Farwick et al., Cosm & Toil, 124(2) : 63-72 ; Masukawa et al., J Lipid Res., 50(8) : 1708-1719, 2009). Le céramide est par exemple choisi dans le groupe constitué par lesdits céramides CER1 à 9.

D'autre part, les présents inventeurs ont montré que les NMF étaient affectés par les conditions déshydratantes. Le facteur naturel d'hydratation, aussi appelé NMF (Natural Moisturizing Factor) est issu de la protéolyse de la filaggrine selon une cascade de réactions impliquant des enzymes dont notamment la caspase 14 et la peptidylarginine deiminase (PAD1). Le NMF est un mélange de substances hygroscopiques ayant la propriété de retenir l'eau (Fluhr et al., Exp Dermatol., 19(6) : 483-492, 2010). Parmi celles-ci, le sel de sodium de l'acide pyrrolidone carboxylique ou PCA Na (provenant de la cyclisation de l'acide glutamique libéré par la décomposition de la profilaggrine) et les lactates sont les substances les plus hygroscopiques. Le NMF comprend en outre des acides aminés libres (sérine, citrulline...), des citrates et des formiates, de l'urée, des ions, de l'azote, de l'acide urique, de la glycosamine, de la créatinine, des phosphates, ainsi que des composés non encore identifiés. La quantité de NMF peut être mesurée par toutes les méthodes connues de l'homme du métier, notamment par microspectroscopie de Raman. A titre illustratif, le marqueur biologique est donc le NMF.

Les inventeurs ont ainsi montré que les marqueurs de l'inflammation sont particulièrement exprimés après que la peau d'enfant a été incubée en atmosphère sèche. L'inflammation est une réaction normale de défense de l'organisme, mais elle peut contribuer à la diminution de l'intégrité de la peau. En outre, les inventeurs ont montré que dans le même temps, la déshydratation induit dans un modèle de peau d'enfant une réduction de l'expression des marqueurs de la fonction barrière et des marqueurs préférentiellement exprimés dans les cellules souches.

Par « marqueurs de l'inflammation cutanée », on entend ici les marqueurs dont la variation d'expression corrèle avec l'inflammation cutanée.

On entend par « inflammation » l'ensemble des mécanismes réactionnels de défense par lesquels l'organisme reconnaît, détruit et élimine toutes les substances qui lui sont étrangères. « L'inflammation cutanée » correspond plus particulièrement à une réaction du système immunitaire en réaction à une agression sur la peau, telle qu'une agression de l'environnement, occasionnant ou non une plaie, voire un dommage vasculaire le cas échéant. L'inflammation cutanée peut se manifester par un érythème, caractérisé par une rougeur associée à une vasodilatation locale, un œdème, caractérisée par un gonflement, et une sensation de chaleur. En outre, l'inflammation cutanée s'accompagne d'une variation de niveau d'expression ou de concentration de marqueur géniques ou protéiques bien connus de l'homme du métier, qui pourra se référer par exemple à Vahlquist (Acta Derm Venereol; 80: 161 ; 2000).

Le déclenchement et la poursuite de l'inflammation, sa diffusion à partir du foyer initial font appel à des facteurs qui sont synthétisés localement ou qui sont à l'état de précurseur inactif dans la circulation. Selon le type de médiateurs synthétisés, on peut différencier des processus particuliers dans la réaction d'inflammation. Ainsi, l'inflammation cutanée regroupe au moins trois processus distincts, l'inflammation protéique, l'inflammation lipidique et l'inflammation neurogène (ou neurogénique).

Par « inflammation protéique » ou « inflammation aspécifique », on entend ici la production en réponse à une agression extérieure de médiateurs inflammatoires protéiques, tels que les cytokines IL-1, IL-2, IL-6, IL8 et TNF$\alpha$, le système du complément, ou les protéines impliquées dans la coagulation, le cas échéant. Par « inflammation lipidique », on entend ici la production à ladite agression extérieure de médiateurs lipidiques, notamment les prostaglandines et les leucotriènes qui sont tous deux synthétisés à partir de l'acide arachidonique, ainsi que l'activation des enzymes responsables de cette production (Shimizu, Annu Rev Pharmacol Toxicol., 49: 123-150, 2009). Les médiateurs protéiques et lipidiques ainsi produits vont induire une cascade de réactions au sein de la peau impliquant d'autres cellules de l'inflammation, en particulier, des cellules immunitaires et vasculaires. Le résultat clinique se traduit notamment par des rougeurs, voire un œdème.

En réponse à une agression extérieure, le système neurosensoriel peut être stimulé et associé à la réaction inflammatoire mettant en œuvre alors d'autres acteurs cellulaires comme les nerfs (ou terminaisons nerveuses) et des cellules telles que le mastocyte. Par « inflammation neurogène », on entend ici la libération par les terminaisons nerveuses, en réponse à une agression extérieure, de médiateurs spécifiques, notamment des neuropeptides (notamment les tachykinines dont la substance P, et le Calcitonin Gene-Related Peptide ou CGRP) ; participe aussi à l'inflammation neurogène selon l'invention, l'activité de récepteurs particuliers tels que la récepteur de la substance P ou le récepteur TRPV1. L'inflammation neurogène résulte le plus souvent en une sensation de de douleur et/ou d'inconfort et/ou de sensation de démangeaison (prurit).

Le marqueur de l'inflammation cutanée est par exemple choisi parmi les marqueurs de l'inflammation protéique, les marqueurs de l'inflammation lipidique et les marqueurs de l'inflammation neurogène.

A titre illustratif, le marqueur de l'inflammation protéique est choisi dans le groupe constitué par les interleukines, de préférence IL1α et IL8. L'interleukine IL1α humaine possède une séquence protéique représentée par la séquence de référence NCBI : NP_000566. Cette protéine est codée par le gène IL1A humain (référence NCBI : Gene ID: 3552), dont la séquence correspond à la référence NCBI : NM_000575. La séquence protéique de l'interleukine IL-8 humaine correspond à la séquence de référence NCBI : NP_000575. Cette protéine est codée par le gène IL8 humain (référence NCBI : Gene ID: 3576). La séquence de celui-ci est accessible sous la référence NCBI : NM_000584.

Le récepteur du facteur de croissance nerveuse NGFR possède une séquence protéique représentée par la séquence de référence NP_002498. Cette protéine est codée par le gène NGFR (référence NCBI : Gene ID: 4804). La séquence de celui-ci est accessible sous la référence NCBI : NM_002507.

Le récepteur OSMR (« Oncostatin-M specific receptor subunit beta » ou « oncostatin M receptor ») est un récepteur de cytokines de type I, dont le ligand est l'oncostatine. La protéine OSMR correspond à la séquence représentée par le numéro d'accession Genbank NP_001161827, et est codée par le gène OSMR (référence NCBI : Gene ID: 9180), de séquence NM_001168355.

La protéine F2RL1 (coagulation factor II (thrombin) receptor-like 1), qui est aussi appelée PAR2 (Protease activated receptor 2) ou GPR11 (G-protein coupled receptor 11) est un récepteur qui est codé par le gène F2RL1 (référence NCBI : Gene ID: 2150) chez l'homme. Ce récepteur, qui fait partie de la famille des récepteurs à 7 domaines transmembranaires ou GPCR, joue un rôle dans la modulation des réponses inflammatoires. La séquence de cette protéine est accessible sous la référence NCBI : NP_005233, tandis que la séquence du gène F2RL1 peut être obtenue avec le numéro d'accession Genbank NM_005242.

Le marqueur de l'inflammation lipidique est choisi avantageusement parmi les prostaglandines, dont en particulier la prostaglandine E2, et les enzymes de la synthèse de celles-ci à partir de l'acide arachidonique, en particulier PTGS2.

La prostaglandine E2 (PGE2), est un dérivé bien connu de l'acide arachidonique obtenu par l'action de la cyclo-oxygénase. Il existe deux isoformes de cyclo-oxygénases (COX) : la cyclo-oxygénase 1 qui est constitutive dans les tissus et la cyclo-oxygénase 2 qui est induite par les phénomènes inflammatoires. Une stimulation pro-inflammatoire (traumatisme, cytokines...) conduit ainsi à la synthèse de PGE 2 qui est responsable d'une vasodilatation (générant rougeur et œdème), d'une sensibilisation des nocicepteurs à la bradykinine et l'histamine (responsables de la douleur) et de la fièvre (avec les cytokines IL1 et IL6).

L'enzyme cyclo-oxygénase 2, aussi appelée prostaglandin-endoperoxide synthase (PTGS), est codée par le gène PTGS2 humain (référence NCBI : Gene ID: 5743). La séquence de ce gène est disponible sous la référence NCBI : NM_000963 et la séquence de la protéine sous la référence NCBI : NP_000954.

A titre d'illustration, le marqueur de l'inflammation neurogène est choisi parmi les neuropeptides et les récepteurs de neuropeptides, notamment les récepteurs TRPV1 et SRP. Le récepteur TRPV1 (Transient Receptor Potential Vanilloid 1) est une protéine membranaire de type canal cationique de la famille des TRP et de séquence de référence NCBI : NP_061197. Au niveau de la peau, TRPV1 est exprimé par les kératinocytes, les mastocytes et les fibres nerveuses. En réponse à un agresseur, l'activation de TRPV1 conduit à la production de cytokines et de neuropeptides et est un acteur de l'inflammation neurogène. Le gène codant le récepteur TRPV1 est le gène TRPV1 (référence NCBI : Gene ID: 7442), dont la séquence a pour référence NCBI : NM_018727. Le gène TRPV3, (référence NCBI : Gene ID: 162514), dont la séquence a pour référence NCBI : NM_001258205, code un récepteur de la même famille que TRPV1. La séquence de ce récepteur TRPV3 correspond à la séquence de référence NP_001245134.

La protachykinin-1 est une protéine qui est codée chez l'homme par le gène TAC1 (référence NCBI : Gene ID: 6863), dont la séquence a pour référence NCBI : NM_003182. La séquence de cette protéine est est accessible sous la référence NCBI : NP_003173. Le récepteur SPR (substance P receptor ; also known as neurokinin 1 receptor, NK1R, or tachykinin receptor 1, TACR1) est un récepteur couplé à la protéine G (GPCR) qui transmet le signal de la substance P (SP) et des autres tachykinines. Il est codé par le gène TACR1 humain dont la séquence a pour référence NCBI : NM_001058. Sa séquence peptidique est la séquence de référence NCBI : NP_001049.

Le récepteur MRGPRD est un récepteur de la famille des récepteurs à 7 domaines transmembranaires, aussi appelés GPCR. Le récepteur MRGD (Mas-related G-protein coupled receptor member D) est plus particulièrement exprimé dans une sous-population de neurones sensoriels qui participent à la détection de la douleur. Ce récepteur est codé par le gène est codée par le gène MRGPRD humain (référence NCBI : Gene ID: 116512). La séquence de ce gène est disponible sous la référence NCBI : NM_198923 et la séquence de la protéine sous la référence NCBI : NP_944605.

Les phospholipases 2A représentent une famille d'enzyme qui libèrent des médiateurs pro-inflammatoires (lysophospholipides, acides gras oxydés) par hydrolyse de phospholipides oxydés à courte chaîne présents sur les lipoprotéines de basse densité (LDL) oxydées. Parmi celles-ci, la Phospholipase A2 Groupe II F est codée par le gène PLA2G2F humain (référence NCBI : Gene ID: 64600). La séquence de ce gène est disponible sous la référence NCBI : NM_022819 et la séquence de la protéine sous la référence NCBI : NP_073730.3

A titre d'exemple, le marqueur de l'inflammation cutanée est donc choisi parmi le groupe consistant en IL1A, IL8, PTGS2, NGFR, TAC1, TAC1R, TRPV1, TRPV3, MRGPRD, OSMR, PLA2G2F et F2RL1.

Les présents inventeurs ont par ailleurs montré que la déshydratation induit une réduction de l'expression des marqueurs de la fonction barrière et des marqueurs préférentiellement exprimés dans les cellules souches.

Les « marqueurs de la barrière » comprennent les marqueurs qui sont spécifiquement exprimés dans les couches de l'épiderme les plus externes et qui participent de la fonction barrière.

Comme il est bien connu de l'homme du métier, la peau a pour fonction principale d'établir une barrière de protection contre les atteintes de l'environnement tout en permettant certains échanges entre le milieu intérieur et le milieu extérieur. Cette fonction barrière est principalement portée par le *stratum corneum* de l'épiderme. Les lipides et les cornéodesmosomes intercellulaires ainsi que l'enveloppe cornée des cornéocytes en sont les constituants clés.

Cependant, sous le *stratum corneum,* les jonctions serrées constituent une seconde ligne de fonction barrière. Ces jonctions constituent au niveau du *stratum granulosum* une barrière de diffusion paracellulaire sélective prévenant de la pénétration de molécules délétères. Les jonctions serrées sont composées de différentes protéines transmembranaires comme notamment les claudines, l'occludine et ZO1.

Les fonctions de barrière portées par le *stratum corneum* et les jonctions serrées sont étroitement liées. En effet, l'altération de l'une ou l'autre peut influencer la formation de l'autre.

A titre illustratif, les marqueurs de la fonction barrière sont des marqueurs exprimés dans le *stratum corneum* ou des marqueurs exprimés dans les jonctions serrées du *stratum granulosum.* Par exemple, ledit marqueur de la barrière épidermique est sélectionné dans le groupe constitué de la desmogléine 1 (DSG), la scielline (SCEL), la peptidyl arginine déiminase 1 (PADI1), la caspase 14 (CASP14), la loricrine (LOR), la transglutaminase 1 (TGM1) et la claudine 1 (CLDN1).

Le cornéodesmosome est la seule structure jonctionnelle de la couche cornée, ce qui souligne l'importance de cette structure pour le maintien de l'intégrité de la couche cornée. La desmogléine 1 est une protéine constitutive des cornéodesmosomes dont la séquence est disponible sous la référence NP_001933. Le gène DSG1 (référence NCBI : Gene ID: 1828) code la desmogléine-1 et possède la séquence de référence NM_001942. La scielline, de séquence NP_001154178, et codée par le gène SC (référence NCBI : Gene ID: 8796) qui a lui-même la séquence NM_001160706 est un précurseur de l'enveloppe cornée. La loricrine est un composant protéique majeur de l'enveloppe cornée, dont elle représente environ 70 % en masse. Cette protéine a pour séquence NP_000418 et est codée par le gène LOR (référence NCBI : Gene ID: 4014) de séquence NM_000427. La formation des liaisons entre les composants protéiques de l'enveloppe de l'enveloppe cornée est assurée par l'enzyme transglutaminase 1. La séquence de cette enzyme correspond à celle qui peut être trouvée sous la référence NP_000350. Le gène TGM1 (référence NCBI : Gene ID: 7051) code la desmogléine-1 et possède la séquence de référence NM_000359.

Les jonctions serrées représentent un des modes d'adhésion cellulaires, dans les tissus épithéliaux, Elles bloquent la circulation de fluides entre les cellules et assurent ainsi l'étanchéité entre deux compartiments tissulaires. Elles sont localisées à l'apex des cellules épithéliales où elles forment une bande continue tout autour qui assure l'étanchéité. Le gène CLDN 1 (référence NCBI : Gene ID: 9076) code la protéine claudine 1 qui est un est un des composants les plus importants des jonctions serrées. Cette protéine a une séquence correspondant à celle dont la référence NCBI est NP_066924. La séquence du gène CLDN1 est accessible sous la référence NM_021101.

Les marqueurs de la barrière comprennent aussi des marqueurs du métabolisme des NMF et des lipides intercornéocytaires. Ces composés permettent de retenir l'eau dans l'épiderme au cours de son ascension vers la couche cornée. La Peptidyl arginine Deiminase 1 (PADI1) et la caspase 14 (CASP14) sont deux enzymes impliquées dans la maturation de la filaggrine qui permet d'obtenir le NMF. La peptidyl -arginine déiminase 1, de séquence NP_037490, est un enzyme qui enlève une fonction imine sur la filaggrine et la kératine K1, qui maintient l'hydratation du stratum corneum, et par conséquent la fonction de barrière cutanée. Cette enzyme est codée par le gène PADI1 dont la séquence (référence NCBI : Gene ID: 29943) peut être trouvée sous la référence NM_013358. La caspase-14 est un membre de la famille des caspases qui est nécessaire pour la dégradation de la filaggrine en NMF (Hoste et al., J Invest Dermatol. 131(11): 2233-2241, 2011). Cette protéine, de séquence NP_036246, est codée par le gène CASP14 (référence NCBI : Gene ID: 23581) dont la séquence se trouve sous la référence NM_012114.

Les « marqueurs préférentiellement exprimés dans les cellules souches » recouvrent les marqueurs, et plus spécifiquement les gènes et les protéines, qui sont spécifiquement présents dans les cellules souches épidermiques.

Par « cellule souche de l'épiderme » ou « cellule souche épidermique », on entend ici, une cellule de l'épiderme capable de renouvellement à long terme. Les cellules souches épidermiques comprennent, entre autres, les cellules souches folliculaires, les cellules souches sébacées et les cellules souches basales, ces dernières étant aussi appelées cellules souches épidermiques interfolliculaires. On entend par « cellules souches folliculaires », « cellules souches sébacées » et « cellules souches basales », les cellules souches situées respectivement dans la région du bulge/renflement du follicule pileux, dans les glandes sébacées et dans la couche basale de l'épiderme.A titre d'exemple, les cellules souches épidermiques de l'invention sont des cellules souches basales.

Plus précisément, on entend par cellule souche épidermique, une cellule dotée d'un potentiel élevé de renouvellement à long terme. Par « potentiel de renouvellement », on entend ici la capacité de subir au moins un cycle de division cellulaire. Un « potentiel élevé de renouvellement à long terme » représente donc la capacité d'une cellule d'entrer dans plusieurs cycles de division cellulaire successifs. Il est bien connu que les cellules différenciées de la peau ne sont pas capables d'effectuer plusieurs divisions successives (Fortunel et Martin, J Soc Biol, 202(1) : 55-65, 2008). Il est entendu ici que « successif » ne signifie pas « consécutif » et qu'il peut exister des périodes pendant lesquelles une cellule souche reste quiescente sans toutefois perdre son potentiel élevé de renouvellement à long terme.

La conservation d'un potentiel élevé de renouvellement à long terme s'exprime par une division asymétrique produisant deux cellules différentes. La première cellule fille est une cellule souche identique à la cellule souche mère, tandis que la seconde est une cellule d'amplification transitoire qui se divise de façon limitée sur une courte période puis entre dans le processus de différenciation. Avantageusement, les cellules souches épidermiques sont donc en outre capables de générer au moins un type de cellule épidermique par différenciation. Autrement dit, la cellule d'amplification transitoire est capable de donner au moins un type de cellule épidermique par différentiation. Par exemple, ladite cellule épidermique est un kératinocyte. A titre illustratif, la cellule d'amplification transitoire est capable de donner tous les types de cellules épidermiques par différentiation.

A titre d'illustration, les marqueurs exprimés dans les cellules souches sont des marqueurs qui participent aux fonctions et à la protection des cellules souches. On citera par exemple les marqueurs ΔNp63, BIRC5 (survivine), FN1 (fibronectin 1), MCSP (Melanoma-associated Chondroitin Sulfate Proteoglycan), LRIG1 (Leucine-rich repeats and immunoglobulin-like domains protein 1), GJA1 (connexin 43), NID1 (nidogen 1), KRT15 (keratin 15), KRT19 (keratin 19), EGFR (epidermal growth factor receptor), CD71 (transferrin receptor), DSG3 (desmoglein 3), ITGB1BP1 (integrin beta1 binding protein), ITGA6 (integrin alpha 6), ITGB1 (integrin beta1) et ITGB4 (integrin beta 4) ou encore des marqueurs impliqués dans la signalisation et la régulation de l'activité des cellules souches comme Wnt/beta catenin, sonic hedgehog (SHH), NOTCH1 (Notch homolog 1, translocation-associated). Les marqueurs ΔNp63 et survivine sont des marqueurs de résistance à l'apoptose, ayant donc un rôle dans la survie des cellules souches. Les cytokératines 15 et 19 sont des marqueurs positifs des cellules souches, la cytokératine 15 étant un marqueur de survie de celles-ci. Le MCSP colocalise avec les intégrines dans les cellules qui ne se divisent pas, tandis que les intégrine beta1 (marqueur d'adhésion à la matrice extracellulaire de la membrane basale) et intégrine alpha 6 (constituant des hemidesmosomes marqueur de liaison des kératinocytes entre eux) sont des protéines de surface qui participent à la communication intercellulaire, régulent les processus de différenciation/prolifération, ainsi que l'interaction avec la niche. Le récepteur à la transférine CD71 est un marqueur de surface connu des cellules souches, qui est utilisé pour isoler, dans une population de cellules integrine-alpha6 positives, les cellules à haut pouvoir clonogéniques. Enfin, Lrig1 est un antagoniste du récepteur à l'Epidermal Growth Factor (EGFR), maintenant ainsi les cellules souches quiescentes, alors que le récepteur EGFR, qui est un marqueur dont l'absence caractérise les cellules souches, entraine au contraire les cellules dans la voie de la prolifération.

A titre illustratif, le marqueur préférentiellement exprimé dans les cellules souches est choisi dans le groupe constitué des marqueurs ΔNp63, KRT15 (keratin 15), KRT19 (keratin 19), BIRC5 (survivine), et NOTCH1 (Notch Homolog 1). Ces marqueurs sont bien connus de l'homme du métier. Les gènes KRT15 (référence NCBI : Gene ID: 3866), KRT19 (référence NCBI : Gene ID: 3880), BIRC5 (référence NCBI : Gene ID: 332) et NOTCH1 (référence NCBI : Gene ID: 4851) correspondent ainsi aux séquences représentées par les numéros d'accession Genbank suivants : NM_002275, NM_002276, NM_001012270, et NM_017617, respectivement. Les protéines keratin 15, keratin 19, survivine et Notch homolog 1 correspondent quant à elles aux séquences représentées par les numéros d'accession Genbank suivants : NP_002266, NP_002267, NP_001012270 et NP_060087, respectivement. La protéine ΔNp63 est une isoforme de la protéine TP63 (Tumor protein 63) obtenue par transcription à partir d'un promoteur interne situé dans un intron. Cette protéine correspond à la séquence représentée par le numéro d'accession Genbank : NP_001108452, et est codée par le gène TP63 (référence NCBI : Gene ID: 8626), dont la séquence est disponible sous le numéro d'accession Genbank suivant : NM_001114980.

Il sera par ailleurs évident à l'homme du métier que la méthode de l'invention permettra une évaluation de l'efficacité de la formulation ou de l'actif d'autant plus complète qu'un grand nombre de marqueurs de types différents seront utilisés.

La méthode ici décrite peut comprendre une étape c) de mesure du niveau d'expression d'une combinaison de marqueurs biologiques. A titre d'exemple, ladite combinaison comprend au moins deux marqueurs, lesdits marqueurs étant choisis dans au moins deux catégories différentes de marqueurs décrits-ci-dessus : NMF, lipides, dont notamment les céramides, marqueurs de l'inflammation, marqueurs de la barrière et marqueurs des cellules souches. Par exemple, ladite combinaison comprend plus de deux marqueurs. A titre illustratif, chacun des marqueurs appartient à une catégorie distincte de marqueurs décrites ci-dessus. Il est aussi possible d'utiliser des combinaisons de marqueur telles que définies ci-dessus, dans lesquelles certaines classes de marqueurs sont représentées par plus d'un marqueur.

L'utilisation de combinaisons de marqueurs comprenant au moins un marqueur de chacun des différents types indiqués plus haut est particulièrement avantageuse.

Pour chacun de ces marqueurs, le terme « niveau d'expression » se réfère à la concentration cellulaire dudit marqueur. Ainsi le niveau d'expression des céramides correspond à la concentration desdits lipides dans la cellule. Si le marqueur est un gène, le « niveau d'expression » correspond à la concentration cellulaire d'au moins un des produits du gène dudit marqueur. Plus précisément, le niveau d'expression dudit marqueur biologique correspond à la quantité ou à la concentration cellulaire du transcrit dudit gène ou de la protéine issue dudit transcrit. A titre d'exemple, le niveau d'expression dudit marqueur biologique correspond à la quantité ou à la concentration cellulaire du transcrit dudit gène. Selon un autre exemple, le niveau d'expression dudit marqueur biologique correspond à la quantité ou à la concentration cellulaire de la protéine issue dudit transcrit.

Par la « mesure du niveau d'expression d'une combinaison de marqueurs biologiques », on entend ici la mesure du niveau d'expression de chacun des marqueurs de la combinaison. L'expression d'un gène peut être mesurée par exemple au niveau nucléotidique, en mesurant la quantité de transcrits dudit gène, et peut aussi être mesurée par exemple au niveau peptidique, en mesurant par exemple la quantité des protéines issus desdits transcrits. Ainsi, par « mesure du niveau d'expression dudit gène » on entend la mesure de la quantité de produit du gène sous sa forme peptidique ou sous sa forme nucléotidique.

De façon générale, l'expression du marqueur biologique sera détectée *in vitro* à partir du modèle de peau reconstruite.

Le procédé ici décrit peut comprendre une ou plusieurs étapes intermédiaires entre l'obtention du modèle de peau reconstruite et la mesure de l'expression du marqueur biologique, lesdites étapes correspondant à l'extraction à partir dudit modèle de peau reconstruite d'un échantillon lipidique, d'un échantillon de NMF, d'un échantillon d'ARNm (ou de l'ADNc correspondant) ou d'un échantillon de protéine. Celui-ci peut ensuite être directement utilisé pour mesurer l'expression du marqueur. La préparation ou l'extraction d'ARNm (ainsi que la rétrotranscription de celui-ci en ADNc), de protéines, de lipides ou de NMF à partir d'un échantillon de cellules de peau ne sont que des procédures de routine bien connues de l'homme du métier.

Une fois qu'un échantillon d'ARNm (ou d'ADNc correspondant) ou de protéine est obtenu, l'expression du marqueur, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible.

Quand l'expression du marqueur est mesurée au niveau de l'ARNm (ou d'ADNc correspondant), n'importe quelle technologie habituellement utilisée par l'homme du métier peut être mise en œuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose.

Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus..). Ces technologies sont utilisées en routine par l'homme du métier et il n'est donc pas besoin de les détailler ici. Des exemples de mises en œuvre de l'invention basées sur l'analyse d'expression génique (puces à ADNc) et sur la PCR quantitative sont décrits dans la section expérimentale.

Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922;

# EP 3 391 046 B1

U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676-684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

Quand l'expression du marqueur est mesurée au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérométrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

Si le marqueur biologique est un lipide, notamment un céramide, l'homme du métier pourra utiliser toutes les méthodes à sa disposition pour mesurer la teneur en lipide dans un échantillon de cellules cutanées. Ces méthodes comprennent, entre autres, la chromatographie liquide en phase gazeuse (HPLC, voir par exemple Sullivan et al., Arch Ophthalmol., 120(12) : 1689-99, 2002), par exemple couplée à un détecteur évaporatif à diffraction de la lumière (HPLC-ESD, voir Nordbäck et al., J. High Resolut. Chromatogr., 22 : 483-486, 1999 ; Torres et al., J. Chromatogr. A., 1078 : 28-34, 2005) ; la chromatographie en couche mince (TLC, par exemple Downing et al., J Invest Dermatol., 77(4) : 358-360, 1981; Nordstrom et al., J Invest Dermatol., 87(2) : 260-263, 1986) ; la résonance magnétique nucléaire (NMR, voir par exemple Robosky et al., J Lipid Res., 49(3) : 686-692, 2008) ; la microspectroscopie confocale in vivo de Raman ; la spectrométrie de masse, la chromatographie en phase gazeuse couplée à la spectrométrie de masse (GC-MS, voir O'Neill et al., J Chromatogr Sci., 14(1) : 28-36, 1976) ; la chromatographie en phase liquide couplée à la spectrométrie de masse (voir par exemple van Smeden et al., J Lipid Res, 52(6):1211-1221, 2011) ; la chromatographie en phase liquide ultra-performante (UPLC, voir Rainville et al., J Proteome Res., 6(2):552-558, 2007 ; Castro-Perez et al., J Proteome Res., 10(9) : 4281-4290, 2011). On peut également analyser l'organisation de ces lipides dans la peau et plus particulièrement dans le *stratum corneum* (ou couche cornée), organisation lamellaire ou latérale, par des techniques par exemple de diffraction aux rayons X (Bouwstra et al., J Invest Dermatol., 97(6): 1005-1012, 1991 ; van Smeden et al., J Lipid Res., 52(6):1211-1221, 1991) ou par spectroscopie infra-rouge à transformée de Fourier (Gorcea et al., Int J Pharm. Nov 10, 2011. ) ou par une analyse morphométrique en microscopie électronique (Daehnhardt-Pfeiffer et al., Skin Pharmacol Physiol., 25(3): 155-161, 2012) ou par une analyse en microscopie électronique de section vitreuse de peau combinée à une analyse moléculaire (Iwai et al., J Invest Dermatol., Apr 26, 2012).

Le dosage du NMF est une procédure bien connue de l'homme du métier. Il est en particulier possible de doser le NMF par l'utilisation de microspectroscopie confocale in vivo de Raman. C'est une procédure couramment utilisée dans le domaine depuis au moins 15 ans. A titre d'exemple, on citera entre autres les publications de Caspers et al. (J Invest Dermatol., 116(3) : 434-442, 2001), Vyumvuhore et al. (J Biomed Opt., 19(11) : 111603, 2014), ou encore Falcone et al. (Skin Pharmacol Physiol, 28 : 307-317, 2015). Il est aussi possible de doser les NMF par la chromatographie en phase liquide couplée à la spectrométrie de masse. On se référera par exemple à Piraud et al. (Rapid Commun Mass Spectrom, 19(12):1587-602, 2005), Petritis et al. (Journal of Chromatography A, 833(2): 147-155, 1999), Henriksen et al. (J Am Soc Mass Spectrom, 16(4): 446-455, 2005) ou encore Yang (Application of biophysics and bioengineering to the assessment of skin barrier function. Thesis (Doctor of Philosophy (PhD)). University of Bath, UK, 2011).

Par « un niveau d'expression de référence d'un marqueur biologique », on entend ici tout niveau d'expression dudit marqueur utilisé à titre de référence. Par exemple, un niveau d'expression de référence peut être obtenu en mesurant le niveau d'expression du marqueur d'intérêt dans un modèle de peau d'enfant, dans des conditions particulières. L'homme du métier saura choisir ces conditions particulières en fonction de son objectif lors de la mise en œuvre du procédé.

Selon un autre exemple, le niveau d'expression de référence d'un marqueur biologique est le niveau d'expression dudit marqueur obtenu dans un modèle de peau d'enfant, mis en contact avec une formulation ou l'actif de référence, et exposé à des conditions de dessiccation.

Lorsque le niveau d'expression de référence est un niveau d'expression obtenu dans un modèle de peau exposé à des conditions de dessiccation, l'homme du métier comprendra aisément que les conditions de dessiccation du modèle de peau utilisé dans le procédé de l'invention et du modèle utilisé pour obtenir un niveau d'expression de référence sont préférentiellement identiques. Ainsi, les conditions de dessiccation utilisées, notamment le taux d'humidité dans l'atmosphère, ainsi que la durée de l'exposition utilisée dans le procédé de l'invention et du modèle utilisé pour obtenir un niveau d'expression de référence sont préférentiellement identiques.

Par exemple, l'homme du métier pourra utiliser comme formulation de référence toute formulation connue de l'art antérieur pour son effet dans la prévention ou le traitement de la peau sèche.

A titre illustratif, la formulation de référence est une émulsion huile-dans-eau, telles que celles décrites ci-dessous. Par exemple, la formulation de référence est la formulation A, laquelle contient des perséoses d'avocat. Ceux-ci sont des sucres en C7 obtenus à partir de l'avocat. Les perséoses d'avocat et leurs procédés d'extraction sont décrits dans, entre autres, WO 2004/112742, WO 2005/105123, WO 2005/115421, WO 2008/025847, WO 2011/073281, WO 2014/017049, WO 2014/122326, WO 2015/044230, etc.

PRODUIT A : EMULSION E/H type Cold Cream

| MP | % |
|---|---|
| AQUA | QSP |
| CAPRYLIC/CAPRIC TRIGLYCERIDE | 5 à 20% |
| COCO-CAPRYLATE/CAPRATE | 1 à 10% |
| POLYGLYCERYL-2-DIPOLYHYDROXYSTEARATE | 1 à 10% |
| GLYCERIN | 1 à 10% |
| CIRES | 1 à 10% |
| HUILE VEGETALE | 1 à 10% |
| POLYGLYCERYL-3 DIISOSTEARATE | 1 à 5% |
| MAGNESIUM SULFATE | 0 à 2% |
| STEARALKONIUM HECTORITE | 0 à 2% |
| PRESERVATIVES | 0 à 2% |
| PERSEA GRATISSIMA FRUIT EXTRACT / AVOCADO PERSEOSE CERAMIDES | 0 à 5% 0 à 1% |

PRODUIT B : EMULSION E/H type Cold Cream

| MP | % |
|---|---|
| AQUA | QSP |
| Huile de paraffine | 1 à 10% |
| Glyceryl Stearate | 1 à 10% |
| Glycerine | 1 à 10% |
| Acide stearique | 1 à 10% |
| Alcool cetylique | 1 à 5% |
| PEG-12 | 1 à 5% |
| Triglycerides | 1 à 5% |
| Huiles végétales | 1 à 5% |
| Cires végétales | 1 à 5% |
| Octyl palmitate | 0.5 à 5% |
| Carbomer | 0.5 à 2% |

(suite)

| MP | % |
|---|---|
| Silicones | 1 à 5% |
| parfum | 0.5 à 2% |
| PRESERVATIVES | 0 à 2% |
| ACTIFS | 0 à 5% |

PRODUIT C : EMULSION E/H type Cold Cream

| MP | % |
|---|---|
| AQUA | QSP |
| Eau thermale | 5 à 10% |
| Huile de paraffine | 1 à 10% |
| Cetyl palmitate | 1 à 10% |
| Glycerine | 1 à 10% |
| Glycols | 1 à 10% |
| Cires minérales | 1 à 10% |
| Huiles minérales | 1 à 10% |
| Cires végétales | 1 à 5% |
| MAGNESIUM SULFATE | 0 à 2% |
| Magnesium stearate | 0 à 2% |
| Sorbitan isosterate | 1 à 5% |
| Polyglyceryl-3 polyricinoleate | 0.5 à 3% |
| PEG-30dipolyhydroxystearate | 0.5 à 3% |
| Silicones | 0.5 à 2% |
| PRESERVATIVES | 0 à 2% |
| ACTIFS | 0 à 5% |

PRODUIT D : EMULSION E/H type Cold Cream

| MP | % |
|---|---|
| AQUA | QSP |
| Eau thermale | 5 à 10% |
| Huile de paraffine | 1 à 10% |
| Glyceryl Stearate | 1 à 10% |
| Alcool cetylique | 1 à 10% |
| Cires minérales | 1 à 10% |
| C-20-40 Pareth 10 | 0.5 à 5% |
| Cetyl phosphate | 0.5 à 5% |

(suite)

| MP | % |
|---|---|
| parfum | 0.5 à 2% |
| PRESERVATIVES | 0 à 2% |
| ACTIFS | 0 à 5% |

PRODUIT E : LAIT AU COLD CREAM

| INCI UE | % INCI |
|---|---|
| AQUA | QSP |
| CAPRYLIC/CAPRIC TRIGLYCERIDE | 5 à 15% |
| GLYCERIN | 2 à 5% |
| HELIANTHUS ANNUUS SEED OIL | 2 à 5% |
| HYDROGENATED COCONUT OIL | 2 à 5% |
| GLYCERYL STEARATE | 1 à 3% |
| CERA ALBA | 1 à 3% |
| 1,2-HEXANEDIOL | 0.5 à 1.5% |
| CETEARETH-20 | 0.5 à 1.5% |
| PARFUM | 0.5 à 1.5% |
| GLYCERYL CAPRYLATE | 0.1 à 0.5% |
| ACRYLATES/C10-30 ALKYL ACRYLATE CROSSPOLYMER | 0.1 à 0.5% |
| CETEARETH-12 | 0.1 à 0.5% |
| CETEARYL ALCOHOL | 0.1 à 0.5% |
| TOCOPHERYL ACETATE | 0.1 à 0.5% |
| SODIUM HYDROXIDE | 0.05 à 0.3% |
| PERSEA GRATISSIMA FRUIT EXTRACT | 0.05 à 0.3% |
| CERAMIDE NP | 0.05 à 0.3% |
| PHYTOSPHINGOSINE | 0.05 à 0.3% |
|  | 100.000000 |

Produit F : Gel lavant au Cold Cream

| INCI UE | % INCI |
|---|---|
| AQUA | QSP |
| GLYCERIN | 2 à 7% |
| COCAMIDOPROPYL BETAINE | 1 à 5% |
| SODIUM MYRETH SULFATE | 1 à 5% |
| COCO-GLUCOSIDE | 1 à 5% |
| PEG-3 DISTEARATE | 0.5 à 2% |

(suite)

| INCI UE | % INCI |
|---------|--------|
| PEG-150 DISTEARATE | 0.5 à 2% |
| GLYCERYL CAPRYLATE | 0.5 à 2% |
| GLYCOL DISTEARATE | 0.5 à 2% |
| PARFUM | 0.5 à 2% |
| POLYQUATERNIUM-10 | 0.1 à 1% |
| CERA ALBA | 0.1 à 1% |
| PRUNUS AMYGDALUS DULCIS OIL | 0.1 à 1% |
| CITRIC ACID | 0.05 à 0.1% |
| CERAMIDE NP | 0.05 à 0.1% |
| PERSEA GRATISSIMA FRUIT EXTRACT | 0.05 à 0.1% |
|  | 100.000000 |

Selon un autre exemple, le niveau d'expression de référence d'un marqueur biologique est le niveau d'expression dudit marqueur obtenu dans un modèle de peau obtenu à partir d'un échantillon cutané provenant d'un enfant, ledit modèle étant non traité avec la formulation ou l'actif d'intérêt, et non exposé à des conditions de dessiccation.

Selon un autre exemple, le niveau d'expression de référence d'un marqueur biologique est le niveau d'expression dudit marqueur obtenu dans un modèle de peau obtenu à partir d'un échantillon cutané provenant d'un enfant, ledit modèle étant non traité avec la formulation ou l'actif d'intérêt, mais exposé à des conditions de dessiccation.

Selon un autre exemple, le niveau d'expression de référence d'un marqueur biologique est le niveau d'expression dudit marqueur obtenu dans un modèle de peau obtenu à partir d'un échantillon cutané provenant d'un enfant, traité avec la formulation ou l'actif d'intérêt, et non exposé à des conditions de dessiccation.

L'homme du métier comprendra en outre aisément que la comparaison de l'étape d) est de préférence effectuée entre des mesures de niveaux d'expression obtenus pour des modèles de peau obtenus à partir d'échantillons cutanés provenant d'enfants, de structures histologiques similaires, voire identiques. Par « structures histologiques similaires », on entend ici que les proportions relatives des types cellulaires compris dans les modèles de peau comparés sont similaires. Ainsi, il est préférable que les proportions relatives des types cellulaires compris dans le modèle de peau de l'étape a) ne diffèrent pas de plus de 5% des proportions relatives des types cellulaires compris dans le modèle de peau utilisé pour l'obtention du niveau d'expression de référence de l'étape d). Par « proportion relative d'un type cellulaire » on entend icile rapport du nombre de cellules correspondant à ce type cellulaire sur le nombre de cellules totales comprises dans le modèle de peau. Ainsi, il est par exemple préférable que la proportion de kératinocytes sur le nombre de cellules totales dans le modèle de peau de l'étape a) ne diffère pas de plus de 5% de la proportion de kératinocytes sur le nombre de cellules totales dans le modèle de peau utilisé pour l'obtention du niveau d'expression de référence de l'étape d). Par « structures histologiques identiques », on entend que les proportions relatives des types cellulaires compris dans les modèles de peau comparés sont identiques. A titre d'exemple, les proportions relatives des types cellulaires compris dans le modèle de peau de mamelon de l'étape a) sont identiques aux proportions relatives des types cellulaires compris dans le modèle de peau utilisé pour l'obtention du niveau d'expression de référence de l'étape d) lorsqu'elles ne diffèrent pas de plus de 0,1%. Avantageusement, la proportion de kératinocytes sur le nombre de cellules totales dans le modèle de peau de l'étape a) ne diffère pas de plus de 0,1% de la proportion de kératinocytes sur le nombre de cellules totales dans le modèle de peau utilisé pour l'obtention du niveau d'expression de référence de l'étape d).

L'homme du métier comprendra tout aussi aisément que la comparaison de l'étape d) est de préférence effectuée entre des mesures de niveaux d'expression obtenus pour des modèles de peau qui sont de taille, de volume ou de poids similaires, voire identiques. Ainsi, il est préférable que la taille, le volume, ou le poids du modèle de peau de l'étape a) ne diffère pas de plus de 5% de la taille, du volume, ou du poids du modèle de peau utilisé pour l'obtention du niveau d'expression de référence de l'étape d). Par exemple, la taille, et le volume et le poids du modèle de peau de l'étape a) ne diffèrent pas de plus de 5% de la taille, du volume et du poids du modèle de peau utilisé pour l'obtention du niveau d'expression de référence de l'étape c). A titre d'illustration, la taille, et le volume et le poids du modèle de peau de l'étape a) ne diffèrent pas de plus de 0,1% de la taille, du volume et du poids du modèle de peau utilisé pour l'obtention du niveau d'expression de référence de l'étape d).

Alternativement, si les modèles de peau diffèrent de plus de 5 % de par la taille, le volume et le poids, l'homme du métier pourra normaliser le niveau obtenu à l'étape c) et le niveau de référence de l'étape d) à l'aide d'un facteur de normalisation.

Ce facteur de normalisation pourra par exemple être un marqueur physique directement accessible tel que la masse de cellules de l'échantillon, ou la masse d'un constituant cellulaire, comme la masse d'ADN cellulaire ou la masse de protéines cellulaires.

Il peut aussi être avantageux d'utiliser comme facteur de normalisation le niveau d'expression d'un gène qui est exprimé au même niveau dans toutes les cellules, ou presque, de l'organisme. En d'autres termes, on utilise par exemple comme facteur de normalisation le niveau d'expression d'un gène de ménage. Selon un autre exemple, le niveau obtenu à l'étape c) et le niveau de référence de l'étape d) sont normalisés en utilisant le niveau d'expression, non pas des gènes de ménage, mais des protéines codées par ceux-ci. Un gène de ménage est un gène exprimé dans tous les types cellulaires, qui code une protéine ayant une fonction de base nécessaire pour la survie de tous les types cellulaires. Une liste de gènes de ménage humains peut être trouvée dans Eisenberg et al. (Trends in Genet, 19: 362-365, 2003). Les gènes de ménage incluent par exemple les gènes RPS28, GAPDH, B2M, TFRC, YWHAZ, RPLO, 18S, GUSB, UBC, TBP, GAPDH, PPIA, POLR2A, ACTB, PGK1, HPRT1, IPO8 et HMBS.

L'homme du métier pourra ainsi aisément évaluer l'efficacité de la formulation d'intérêt en fonction de la comparaison de l'étape d).

Selon un aspect ne faisant pas partie de l'invention, il est ici décrit un kit pour la mise en œuvre d'une méthode selon l'invention, comprenant les moyens nécessaires à la mesure du niveau d'expression d'au moins un marqueur choisi parmi les lipides, le NMF, les marqueurs de l'inflammation cutanée, les marqueurs de la fonction barrière et les marqueurs préférentiellement exprimés dans les cellules souches. De préférence, le lipide est un céramide, le marqueur de la fonction barrière est choisi parmi DSG, SCEL, PADI1, CASP14, LOR, TGM1 et CLDN1, et le marqueur préférentiellement exprimé dans les cellules souches est choisi parmi ΔNp63, KRT15, KRT19, BIRC5, et NOTCH1.

En particulier, le kit comprend en outre les moyens nécessaires à la mesure du niveau d'expression d'une combinaison de marqueurs biologiques choisie parmi le groupe comprenant ou consistant en :

- au moins un marqueur de l'inflammation cutanée et au moins un marqueur de la barrière tels que définis plus haut ; ou
- au moins un marqueur de l'inflammation cutanée et au moins un marqueur préférentiellement exprimé dans les cellules souches, tels que définis plus haut ; ou
- au moins un marqueur de la barrière et au moins un marqueur préférentiellement exprimé dans les cellules souches, tels que définis plus haut.

A titre d'exemple, ladite combinaison comprend au moins un marqueur de l'inflammation cutanée et au moins un marqueur de la barrière et au moins un marqueur préférentiellement exprimé dans les cellules souches, tels que définis plus haut.

Les exemples suivants sont fournis ici à titre d'illustration et ne sont pas, sauf indication contraire, destinés à être limitatifs.

DESCRIPTION DES FIGURES

La figure 1 montre la quantification des NMFs dans les panels Peau Normale et Peau Sèche, ainsi que l'évolution des quantités de NMFs en fonction des groupes d'âge.

La figure 2 montre la quantification des céramides dans les panels Peau Normale et Peau Sèche, ainsi que l'évolution des quantités de céramides en fonction des groupes d'âge.

EXEMPLES

**Exemple 1 : <u>Caractérisation de la peau sèche du bébé et de l'enfant <em>in vivo</em></u>**

Afin de caractériser la peau sèche du bébé et de l'enfant, une étude clinique exploratoire a été réalisée.

**1. Matériel et méthodes**

Une étude clinique sous contrôle pédiatrique et dermatologique a été conduite sur deux panels (panel Peau Normale et panel Peau Sèche ; classification après examen clinique réalisé par l'investigateur) de 40 sujets chacun, répartis selon 4 groupes d'âge :
Groupe 1 : 1-28 jours ; Groupe 2 : 3-6 mois ; Groupe 3 : 1 an ; Groupe 4 : 2-4 ans (respectivement Gpe 1, Gpe 2, Gpe 3, Gpe 4).

Des prélèvements biologiques ont été collectés sur l'avant-bras des sujets à l'aide d'écouvillons. Le dosage des NMFs (Facteurs Naturels d'Hydratation) et des Céramides a été réalisé sur les prélèvements collectés par chromatographie liquide couplée à la spectrométrie de masse (LC/MS) pour les céramides ou par chromatographie liquide couplée à une détection UV (LC/UV) pour les NMFs.Analyse des céramides

La présence de céramides présentant une base sphingoïde de type shingosine [S], dihydrosphingosine [DS] et phytosphingosine [P] avec une longueur de chaine paire allant de 16 à 22 atomes de carbone a été recherchée par une méthode LC/MS.

La teneur en céramides a été normalisée par la quantité de protéines totales (dosage BCA).

Analyse des éléments du NMF

Les catabolites de la filaggrine ont été dosés par une méthode LC/UV permettant de cribler l'acide urocanique (UCA) sous ses deux isomères (cis et trans) ainsi que l'acide L-pyrrolidone carboxylique (PCA).

La teneur en NMF a été normalisée par la quantité de protéines totales (dosage BCA).

## 2. Résultats et conclusion

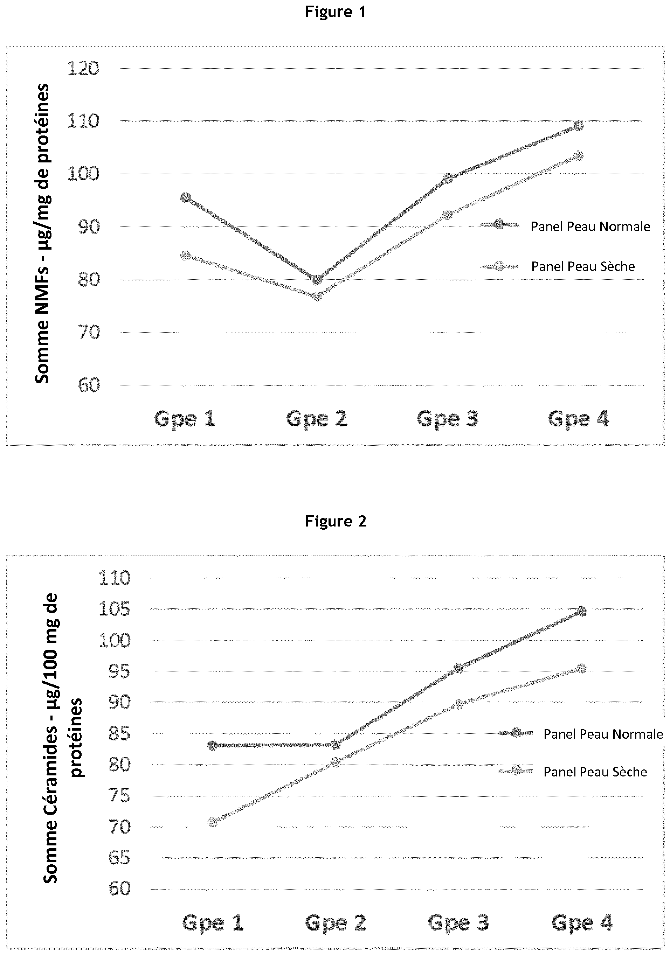

Les résultats présentés ci-dessous montrent que les sujets du panel Peau Sèche présentent des quantités en NMFs significativement plus faibles que ceux du panel Peau Normale, quel que soit le groupe d'âge (figure 1, tableau 1). Ces observations montrent que de faibles taux en NMFs constituent un marqueur biochimique de la peau sèche chez les nouveau-nés, les nourrissons et les jeunes enfants.

D'autre part, les quantités en céramides sont plus faibles pour les sujets du panel à Peau Sèche que pour les sujets du panel Peau Normale (figure 2 , tableau 1), montrant que les céramides constituent également un marqueur de la condition de la peau sèche chez les nouveau-nés, nourrissons, et les enfants qui présentent une peau cliniquement sèche.

En conclusion, la quantité de NMFs et de céramides est diminuée dans la peau d'enfant sèche comparativement à la peau d'enfant normale.

*Tableau 1 : Contenu global en NMFs et céramides pour l'ensemble des panels Peau Normale et Peau Sèche - test t de Student*

|  | Peau Normale | Peau Sèche | Comparaison Peau Sèche vs Peau Normale |
|---|---|---|---|
| Somme des NMFs ($\mu$g/mg de protéines) | 97,37 | 89,43 | -8,2% p<0,05 |
| Somme des Céramides ($\mu$g/100mg de protéines) | 92,68 | 84,44 | -8,9% p<0,05 |

**Exemple 2 : Validation du modèle *in vitro* de « Peau Sèche »**

Afin de valider la modélisation d'un phénotype de peau sèche, la production de NMF (Natural Moisturizing Factor) et de céramides par les épidermes incubés en atmosphère sèche a été évaluée.

## 1. Matériel et méthodes

Les épidermes reconstruits ont été réalisés avec les kératinocytes d'un donneur de 1 an.

La reconstruction des épidermes a été réalisée selon le modèle dérivé de la méthode de Poumay et al (Arch Dermatol Res 2004 ; 296: 203-11). Après 2 jours de culture en immersion, les épidermes reconstruits ou RHE (Reconstructed Human Epidermis) ont été cultivés à l'interface air/liquide pendant 11 jours.

A J11, les épidermes ont été incubés pendant 48h en étuve humide pour les épidermes contrôles (condition normale : 37°C, 5% de CO2 et humidité relative >99%) ou en étuve sèche (37°C, 5% de CO2 et humidité relative <25%).

A l'issue de l'incubation, les quantités de NMF et de céramides produites par les épidermes ont été évaluées.

Les expérimentations ont été répétées 3 fois ; pour chacun de ces 3 essais, 3 réplicats ont été réalisés et analysés.

Analyse des céramides

Les lipides épidermiques ont été extraits par agitation des épidermes à partir d'un mélange de solvants organiques durant 2h à température ambiante. Un traitement par extraction de type solide/liquide a ensuite été réalisé afin d'isoler les céramides des autres lipides constitutifs des épidermes.

Les teneurs en céramides ont été analysées par LC/MS comme décrit dans l'exemple 1.

Analyse des éléments du NMF

Les épidermes reconstruits ont été extraits sous agitation 2h à température ambiante à partir d'un mélange aqueux en présence d'un tensioactif non ionique pour favoriser l'extraction des marqueurs d'intérêt.

Les teneurs en MNF ont été analysées par LC/UV comme décrit dans l'exemple 1.

## 2. Résultats et conclusion

Les céramides sont des composants structurels importants de l'épiderme. Ils sont un des constituants de la matrice lipidique du *stratum corneum.* Le rôle de cette matrice est primordial dans la régulation de la perméabilité hydrique. Elle constitue en effet une barrière hydrophobe qui régule la circulation de l'eau à travers le SC. La matrice lipidique est composée d'un mélange équimolaire de céramides (45 à 50% du poids total), de cholestérol (20-25%) et d'acides gras libres (10-15%). Les céramides sont issues de la transformation de la sphingomyéline par les sphingomyélinases et des glucocéramides par la β-glucocérébrosidase.

La fonction majeure du NMF est de maintenir un niveau d'eau optimal dans le *stratum corneum.* Une source bien connue des constituants du NMF est la filaggrine.

L'incubation des épidermes reconstruits de 1 an en étuve sèche a induit une inhibition significative de la teneur en céramides et en NMF dans les épidermes (Tableau 2).

La diminution de la quantité de NMF et de céramides est une des caractéristiques principales de la peau sèche, connue et décrite dans la littérature. En outre, l'exemple 1 montre que la quantité de NMF et de céramides diminue également dans la peau sèche d'enfant. Le modèle mis en place consistant en l'incubation d'épidermes reconstruits en atmosphère sèche est donc bien représentatif du phénotype de sécheresse cutanée de la peau d'enfant observé *in vivo.*

*Tableau 2 : Teneur en NMF et céramides dans les épidermes reconstruits de 1 an incubés en condition normale (contrôle) ou en étuve sèche (atmosphère sèche)*

| | Epidermes contrôles | Epidermes en atmosphère sèche | Inhibition | Significativité (test t de Student) |
|---|---|---|---|---|
| Teneur en NMF (μg/mg de protéines) | 17,05±0,21 | 12,44±0,82 | -27% | P<0,001 |
| Teneur en céramides (u.a./mg de protéines) | 173,6±3,86 | 149,4±2,62 | -14% | P<0,001 |

## Exemple 3 : Evaluation de l'effet de la délipidation sur la quantité de céramides dans des épidermes reconstruits

Afin de déterminer si d'autres modèles *in vitro* de peau sèche permettent de reproduire le phénotype de la peau sèche d'enfant, le modèle d'induction de peau sèche par délipidation a été testé. La quantité de céramides, un des marqueurs spécifiques de la peau sèche d'enfant, a été analysé dans ce modèle.

Des épidermes reconstruits ont été réalisés selon le modèle dérivé de la méthode de Poumay et al (Arch Dermatol Res 2004 ; 296: 203-11). Après 2 jours de culture en immersion, les épidermes reconstruits ou RHE (Reconstructed Human Epidermis) ont été cultivés à l'interface air/liquide pendant 10 jours.

A J10, les épidermes ont été traités par application topique d'un mélange éther/acétone 1:1 à 30% afin d'induire une altération de la barrière par délipidation, puis incubés pendant 48h (Miyamoto et al., 2002, Akiyama et al., 2010, Valvetcha et al., 2015).

A l'issue de l'incubation, les quantités de céramides produites par les épidermes ont été évaluées.

Analyse des céramides

Les lipides épidermiques ont été extraits par agitation des épidermes à partir d'un mélange de solvants organiques durant 2h à température ambiante. Un traitement par extraction de type solide/liquide a ensuite été réalisé afin d'isoler les céramides des autres lipides constitutifs des épidermes.

La présence de céramides présentant une base sphingoïde de type shingosine [S], dihydrosphingosine [DS] et phytosphingosine [P] avec une longueur de chaine paire allant de 16 à 22 atomes de carbone a été recherchée par une

méthode LC/MS.

<u>Résultats et conclusion</u>

La délipidation des épidermes par le traitement éther/acétone n'induit pas de diminution de la quantité de céramides produite par les épidermes (tableau 3).

Ainsi, de manière surprenante, la délipidation ne permet pas de modéliser le phénotype de peau sèche démontré *in vivo.* Seul le modèle « peau sèche » obtenu par incubation en étuve à atmosphère sèche conçu par les présents Inventeurs et décrit dans l'exemple 2 ci-dessus permet de reproduire le phénotype observé *in vivo* pour la peau sèche d'enfant.

*Tableau 3 : Contenu global en céramides pour les épidermes contrôles et les épidermes délipidés - test t de Student*

|  | Epiderme contrôle | Epiderme délipidé | Comparaison Epiderme délipidé vs Epiderme contrôle |
|---|---|---|---|
| Céramides (Unité arbitraire) | 234,3 ± 26,0 | 227,9 ± 3,9 | -2,8% ns |

**Exemple 4 : Caractérisation de la réponse d'épidermes de nourrisson comparativement à des épidermes adultes**

**1. Matériel et méthodes**

Des épidermes reconstruits ont été réalisés, comme décrit plus haut, avec les kératinocytes d'un donneur de 1 an d'une part et avec les kératinocytes d'un donneur de 19 ans d'autre part ; les kératinocytes de ces deux donneurs provenant d'un prélèvement préputial.

A J10, les épidermes ont été incubés pendant 24h à 48h en étuve humide pour les épidermes contrôles (condition normale : 37°C, 5% de CO2 et humidité relative >99%) ou en étuve sèche (37°C, 5% de CO2 et humidité relative <25%).

Après 24h d'incubation, l'expression génique des marqueurs de l'inflammation, de l'hydratation, de la fonction barrière et des cellules souches a été évaluée par qRT-PCR (PCR quantitative en temps réel).

Après 48h d'incubation, la viabilité et la morphologie des épidermes ont été étudiées.

<u>Evaluation de la viabilité des épidermes reconstruits</u>

La viabilité des épidermes a été évaluée par un test de réduction du MTT en n=2 : les épidermes ont été incubés en présence de MTT (sel de tétrazolium) dont la transformation en cristaux bleus de formazan est proportionnelle à l'activité de la succinate déshydrogénase (enzyme mitochondriale). Après dissociation des cellules et solubilisation du formazan par ajout d'isopropanol/HCL, la densité optique (DO), représentative du nombre de cellules vivantes et de leur activité métabolique, a été mesurée à 540 nm.

<u>Evaluation de la morphologie des épidermes reconstruits</u>

La morphologie des épidermes reconstruits a été évaluée en n=2 par analyse histologique après coloration Hématoxyline / Eosine.

Les épidermes ont été fixés et inclus en paraffine, des coupes transversales ont été réalisées au microtome. Les coupes ont été déparaffinées puis colorées en hématoxyline/éosine. L'hématoxyline colore les noyaux des cellules en bleu/violet ; l'éosine colore les cytoplasmes en rose plus ou moins vif.

L'analyse des coupes histologiques en microscopie a permis d'évaluer et de quantifier certains paramètres morphologiques comme l'épaisseur de la couche de cellules vivantes.

<u>Analyse de l'expression différentielle des gènes</u>

L'expression des marqueurs a été évaluée par qRT-PCR sur les ARN messagers extraits des RHE de chaque traitement.

L'analyse de l'expression des gènes a été réalisée en n=2 à l'aide d'un PCR array contenant 30 gènes d'intérêt et 2 gènes de référence (housekeeping genes). Les ARN totaux de chaque échantillon ont été extraits à l'aide de TriPure Isolation Reagent® selon le protocole préconisé par le fournisseur. La quantité et la qualité des ARN ont été évaluées par électrophorèse capillaire (Bioanalyzer, Agilent). Les ADN complémentaires (ADNc) ont été synthétisés par transcription inverse des ARN en présence d'oligo(dT) et de l'enzyme « transcriptor Reverse Transcriptase ». L'ADNc obtenu a été quantifié par spectrophotométrie, puis les quantités d'ADNc ont été ajustées.

Les réactions de PCR ont été réalisées par PCR quantitative avec le système « light cycler » (Roche Molecular System Inc) et selon la procédure recommandée par le fournisseur. Le mélange réactionnel pour chaque échantillon a été : ADNc, amorces des différents marqueurs utilisés, mélange réactionnel contenant l'enzyme taq DNA polymérase, le marqueur SYBR Green I (agent intercalant de l'ADN) et du MgCl2.

L'incorporation de fluorescence dans l'ADN amplifié est mesurée en continu au cours de cycles de PCR.

L'analyse quantitative des résultats est basée sur le recueil des cycles seuils (ou Ct pour threshold cycle). Le cycle seuil correspond au point où le signal d'émission de fluorescence est statistiquement et significativement plus élevé que le bruit de fond. Le cycle seuil est directement corrélé au nombre de copies initiales de l'ADN cible.

Le tableau 4 liste les gènes qui ont été étudiés.

La valeur d'expression génique relative (ER) est exprimée en unités arbitraires selon la formule suivante :

$$ER = (1/2^{nombre\ de\ cycles}) \times 10^6$$

*Tableau 4 : Classification et nom des gènes étudiés*

| Nom du cluster | Abréviation | Nom du gène |
|---|---|---|
| Housekeeping | RPS28 | Ribosomal protein 28S |
| | GADPH | Glyceraldehyde-3-phosphate dehydrogenase |
| Inflammation aspécifique et neurogène | IL1A | Interleukine 1 alpha |
| | IL8 | Interleukine 8 |
| | PTGS2 | Prostaglandine synthase 2 |
| | NGFR | Recepteur du Nerve Growth Factor |
| | PLA2G2F | Phospholipase A2 Groupe IIF |
| | TAC1 | Tachykinin precursor 1, ou Substance P |
| | TAC1R | Tachykinin receptor 1, ou récepteur à la Substance P |
| | TRPV1 | Transient receptor potential cation channel, subfamily V, member 1 |
| | TRPV3 | Transient receptor potential cation channel, subfamily V, member 3 |
| | MRGPRD | MAS related GPR family member D |
| | F2RL1 | Coagulation factor II (thrombin) receptor-like 1 ou Proteinase Activated Receptor 2 (PAR2) |
| | OSMR | Oncostatin M receptor, récepteur de l'interleukine 31 |
| Différenciation épiderme, Fonction barrière, Hydratation | DSG | Desmogléine 1 |
| | SCEL | Scielline |
| | PADI1 | Peptidyl Arginine Deiminase 1 |
| | CASP14 | Caspase 14 |
| | LOR | Loricrine |
| | TGM1 | Transglutaminase 1 |
| | CLDN1 | Claudine 1 |
| Cellules souches | TP63 | Tumor Protein P63 |
| | KRT15 | Keratin15 |
| | KRT19 | Keratin 19 |
| | BIRC5 | Baculoviral IAP repeat-containing 5 or surviving (=Survivine) |
| | NOTCH1 | Notch Homolog 1 |

**2. Résultats**

**a. Morphologie et viabilité des épidermes**

L'incubation en atmosphère sèche n'a pas fortement altéré la morphologie des épidermes observée en microscopie après coloration en hématoxyline/éosine (données non montrées) ; par ailleurs, la viabilité des épidermes a été impactée de la même manière dans les épidermes 1 an et dans les épidermes adultes (respectivement 30% et 29% d'inhibition, tableau 5). Toutefois, la mesure de l'épaisseur de la couche de cellules vivantes a été plus fortement impactée dans les épidermes de 1 an (-18%) par rapport aux épidermes adultes dans lesquels aucune diminution n'est observée sur ce paramètre (tableau 5).

Ces observations tendent à montrer une sensibilité accrue des épidermes de 1 an face au stress déshydratant.

*Tableau 5 : Analyse de la viabilité et de l'épaisseur de la couche de cellules vivantes*

|  | Epiderme | Contrôle | Atmosphère sèche |
|---|---|---|---|
| **Viabilité (MTT)** | 1 an | 100% | 70% |
|  | Adulte | 100% | 71% |
| **Epaisseur de la couche de cellules vivantes** | 1 an | 100% | 82% |
|  | Adulte | 100% | 100% |

**b. Analyse de l'expression des gènes marqueurs de l'inflammation aspécifique et neurogène**

L'incubation en atmosphère sèche a induit une augmentation du niveau d'expression des marqueurs de l'inflammation aspécifique et neurogène, que ce soit dans les épidermes reconstruits de 1 an ou dans les épidermes reconstruits adultes (tableau 6).

Cette stimulation est cependant beaucoup plus importante dans le cas des épidermes reconstruits de nourrisson (+355%) par rapport aux épidermes adultes (+114%).

Ceci suggère une sensibilité accrue des épidermes de nourrisson dont la réponse inflammatoire et neuro-sensorielle est exacerbée en réponse au stress déshydratant.

*Tableau 6 : Niveau d'expression génique des marqueurs de l'inflammation (expression relative en % par rapport au Contrôle cultivé en conditions normales)*

|  | Epidermes 1 an contrôles | Epidermes 1 an en atmosphère sèche | Epidermes adultes contrôles | Epidermes adultes en atmosphère sèche |
|---|---|---|---|---|
| IL1A | 100 | 177 | 100 | 155 |
| IL8 | 100 | 314 | 100 | 147 |
| PTGS2 | 100 | 1421 | 100 | 666 |
| NGFR | 100 | 223 | 100 | 100 |
| TRPV1 | 100 | 675,5 | 100 | 125 |
| TRPV3 | 100 | 606 | 100 | 150 |
| MRGPRD | 100 | 246 | 100 | 351 |
| F2RL1 | 100 | 171 | 100 | 119 |
| OSMR | 100 | 263 | 100 | 113 |
| **Moyenne d'expression des marqueurs de l'inflammation (%)** | **100** | **455** | **100** | **214** |
| ***Augmentation par rapport au contrôle (%)*** | **-** | ***+355%*** | **-** | ***+114%*** |

**c. Analyse de l'expression des gènes marqueurs des cellules souches**

Les cellules souches de tissus en renouvellement permanent sont classiquement définies comme étant des cellules rares et relativement quiescentes. Elles possèdent une capacité unique d'auto-renouvellement et de régénération tissulaire leur permettant d'assurer l'homéostasie et l'intégrité du tissu dans lequel elles résident.

Parmi les cellules souches de l'épiderme, les cellules souches interfolliculaires situées dans la couche basale constituent le réservoir épidermique principal en cellules souches. Ces cellules résident dans un microenvironnement anatomique et fonctionnel, la niche, qui contribue à maintenir leurs caractéristiques, notamment quand les conditions physiologiques changent. Les cellules souches interfolliculaires et leurs niches sont impliquées dans le maintien de l'intégrité et la régénération de l'épiderme. Les cellules souches ne sont identifiables qu'en suivant plusieurs marqueurs. Nous avons ainsi évalué le niveau d'expression de différents marqueurs géniques caractéristiques des cellules souches dans le modèle de « peau sèche ».

L'incubation en atmosphère sèche a induit une diminution importante (-45%) du niveau d'expression moyen du pool de marqueurs de cellules souches étudié (tableau 7) dans les épidermes de 1 an, alors qu'aucune inhibition de l'expression de ces marqueurs n'a été observée dans les épidermes adultes (+12%).

Ceci tend à montrer une vulnérabilité accrue de ce capital cellulaire dans les épidermes de 1 an.

*Tableau 7 : Niveau d'expression génique des marqueurs de cellules souches (expression relative en % par rapport au Contrôle 1 an)*

| | Epidermes 1 an contrôles | Epidermes 1 an en atmosphère sèche | Epidermes adultes contrôles | Epidermes adultes en atmosphère sèche |
|---|---|---|---|---|
| KRT19 | 100 | 65 | 84 | 120 |
| BIRC5 | 100 | 64 | 85 | 94 |
| TP63 | 100 | 13 | 86 | 80 |
| NOTCH1 | 100 | 77 | 45 | 43 |
| **Moyenne d'expression du pool de marqueurs de Cellules Souches (%)** | **100** | **55** | **75** | **84** |
| ***Modulation par rapport au contrôle (%)*** | **-** | ***-45%*** | **-** | ***+12%*** |

**d. Analyse de l'expression des gènes marqueurs de la barrière et de l'hydratation**

L'incubation en atmosphère sèche a induit une diminution du niveau d'expression des marqueurs de la barrière, que ce soit dans les épidermes reconstruits de 1 an ou dans les épidermes reconstruits adultes (tableau 8).

Cette inhibition est cependant plus importante dans le cas des épidermes reconstruits de nourrisson (-57%) par rapport aux épidermes adultes (-38%).

Ceci suggère une vulnérabilité plus importante des marqueurs de la fonction barrière dans les épidermes de nourrisson.

*Tableau 8: Niveau d'expression génique des marqueurs de la barrière et de l'hydratation (expression relative en % par rapport au Contrôle 1 an)*

| | Epidermes 1 an contrôles | Epidermes 1 an en atmosphère sèche | Epidermes adultes contrôles | Epidermes adultes en atmosphère sèche |
|---|---|---|---|---|
| DSG1 | 100 | 56 | 153 | 92 |
| SCEL | 100 | 20 | 62 | 34 |
| PADI1 | 100 | 51 | 117 | 78 |
| CASP14 | 100 | 47 | 112 | 74 |
| **Moyenne d'expression des marqueurs barrière (%)** | **100** | **43** | **111** | **69** |

(suite)

|  | Epidermes 1 an contrôles | Epidermes 1 an en atmosphère sèche | Epidermes adultes contrôles | Epidermes adultes en atmosphère sèche |
|---|---|---|---|---|
| *Inhibition par rapport au contrôle (%)* | - | *-57%* | *-* | *-38%* |

La **desmogléine 1** (DSG1) est une protéine constitutive des cornéodesmosomes qui assurent la cohésivité des cornéocytes au sein de la couche cornée.

La **scielline** (SCL) est un précurseur de l'enveloppe cornée.

La **PADI1** (Peptidyl arginine Deiminase 1) et la **caspase 14** (CASP14) sont deux enzymes impliquées dans le processing de la filaggrine pour obtenir le NMF.3. Conclusion

L'analyse comparative du comportement des épidermes reconstruits de 1 an et d'adulte (19 ans) dans le modèle d'induction de la peau sèche montre une plus forte vulnérabilité et susceptibilité des épidermes de nourrisson au stress déshydratant induit par l'incubation en atmosphère sèche.

Cette vulnérabilité accrue justifie le développement de produits cosmétiques pour la peau sèche spécifiquement adaptés à la peau du bébé.

**Exemple 5 : Évaluation de produits pour la peau sèche**

Le modèle de peau sèche du bébé mis en place précédemment, consistant à incuber des épidermes reconstruits de nourrisson de 1 an en atmosphère sèche a été utilisé afin d'évaluer comparativement l'efficacité biologique de 4 produits cosmétiques destinés aux peaux sèches.

**1. Matériel et méthodes**

Des épidermes reconstruits ont été réalisés, comme décrit précédemment, à partir de kératinocytes d'un donneur de 1 an.

A J11, les épidermes ont été traités par application topique des produits à l'essai à raison de 5 mg/cm$^2$ puis incubés pendant 6h, 24h ou 48h en étuve sèche (condition atmosphère sèche : 37°C, 5% de $CO_2$ et humidité relative <25%), sauf pour les épidermes contrôles en étuve humide (37°C, 5% de $CO_2$ et humidité relative >99%) et analysés comme décrit plus haut :
Après 6h et 24h d'incubation, l'expression génique des marqueurs de l'inflammation aspécifique et neurogène, de l'hydratation, de la fonction barrière et des cellules souches (tableau 1) a été évaluée par qRT-PCR (PCR quantitative en temps réel) en n=2.

Après 48h d'incubation, la viabilité (MTT) et la morphologie (histologie, coloration hématoxylline/éosine) des épidermes ont été étudiées en n=2 et la production de NMF et céramides analysée en n=3.

Produits à l'essai =formulations de référence telles que citées plus haut :

- Produit A

- Produit B

- Produit C

- Produit D

**2. Résultats**

**a. Morphologie et viabilité des épidermes**

Tout comme lors de l'étape précédente de mise au point du modèle, l'incubation en atmosphère sèche n'a pas altéré significativement la morphologie et la viabilité des épidermes (tableau 9).

*Tableau 9 : Analyse de la viabilité des épidermes*

|  | Viabilité (MTT) |
|---|---|
| **Epiderme contrôle** | 100% |
| **Témoin étuve sèche** | 87% |
| **Produit A** | 90% |
| **Produit B** | 89% |
| **Produit C** | 75% |
| **Produit D** | 97% |

**b. Analyse de l'expression des gènes marqueurs de l'inflammation neurogène et aspécifique**

Les produits A, B et C inhibent les marqueurs d'inflammation exprimés dans les conditions d'incubation en atmosphère sèche (Tableau 10). Ces produits offrent donc une protection contre la réactivité cutanée qui peut être exacerbée dans ces conditions. Seul le produit D n'exerce aucun effet inhibiteur sur ces marqueurs.

*Tableau 10 : Niveau d'expression génique des marqueurs de l'inflammation neurogène et aspécifique*

| (expression relative en % par rapport au témoin étuve sèche) | | | | | |
|---|---|---|---|---|---|
|  | PLA2G2F | TAC1 | TAC1R | **Moyenne d'expression des marqueurs (%)** | *Inhibition par rapport au témoin étuve sèche* |
| Témoin étuve sèche | 100 | 100 | 100 | **100** | |
| Produit A | 90,5 | 81,5 | 70 | **81** | **-19%** |
| Produit B | 75,5 | 66 | 55 | **65** | **-35%** |
| Produit C | 103,5 | 75,5 | 75 | **85** | **-15%** |
| Produit D | 87,5 | 95,5 | 105,5 | **96** | **-4%** |

**c. Analyse de l'expression des gènes marqueurs des cellules souches**

Seul le produit A restaure complètement (96% de restauration) le niveau d'expression des marqueurs des cellules souches inhibés par l'incubation en atmosphère sèche avec une efficacité supérieure aux autres produits testés (Tableau 11).
La produit A protège donc le capital cellulaire dans le modèle de peau sèche.

*Tableau 11 : Niveau d'expression génique des marqueurs de cellules souches (expression relative en % par rapport au Contrôle 1 an)*

|  | KRT15 | KRT19 | BIRC5 | TP63 | **Moyenne d'expression du pool de marqueurs de cellules souches (%)** | *% de restauration* |
|---|---|---|---|---|---|---|
| Contrôle | 100 | 100 | 100 | 100 | **100** | |
| Témoin étuve sèche | 25 | 74 | 45 | 47 | **48** | |
| Produit A | 97 | 130 | 78 | 90 | **98** | *96%* |
| Produit B | 80 | 68 | 32 | 62 | **61** | *61%* |
| Produit C | 86 | 131 | 21 | 70 | **77** | *77%* |
| Produit D | 86 | 74 | 19 | 71 | **63** | *63%* |

**d. Analyse de l'expression des gènes marqueurs de la barrière**

En condition d'incubation en atmosphère sèche, les produits A et B ont rétabli le niveau d'expression des gènes marqueurs de la barrière et montrent une efficacité de restauration (respectivement 140% et 113%) supérieure aux autres formules testées (Tableau 12).

Ainsi, le produit A, et dans une moindre mesure le produit B, protègent l'expression des marqueurs de la barrière altérés par l'incubation en atmosphère sèche.

*Tableau 12 : Niveau d'expression génique des marqueurs de la barrière (expression relative en % par rapport au Contrôle 1 an)*

| | LOR | TGM1 | CLDN1 | DSG1 | Moyenne d'expression des marqueurs de la barrière (%) | % de restauration |
|---|---|---|---|---|---|---|
| Contrôle | 100 | 100 | 100 | 100 | **100** | |
| Témoin étuve sèche | 88 | 93 | 93 | 68 | **85** | |
| Produit A | 103 | 109 | 111 | 103 | **106** | *140* |
| Produit B | 97 | 109 | 110 | 91 | **102** | *113* |
| Produit C | 78 | 91 | 92 | 114 | **94** | *60* |
| Produit D | 72 | 87 | 86 | 101 | **87** | *13* |

La **loricrine** (LOR) est un marqueur tardif de la différenciation épidermique impliqué dans la formation de l'enveloppe cornée.

La **transglutaminase 1** (TGM1) enzyme responsable du cross-linking de différentes protéines pour la mise en place de l'enveloppe cornée, assurant l'étanchéité/solidité du stratum corneum.

La **claudine 1** (CLDN1) est une des protéines constitutives des jonctions serrées, impliquées dans la fonction barrière de la peau, contrôlant notamment les flux hydriques.

La **desmogléine 1** (DSG1) est une protéine constitutive des cornéodesmosomes qui assurent la cohésivité des cornéocytes au sein de la couche cornée.

La **cornéodesmosine** est une protéine constitutive des cornéodesmosomes, jonctions qui se forment entre les cornéocytes pour maintenir la structure du stratum corneum et ainsi participer à une bonne desquamation de la peau [10].

### e. **Analyse de la production de NMF et de céramides**

Les produits A et B ont permis de restaurer la production de céramides et de NMF inhibées dans le modèle de peau sèche (tableau 13) tandis que les deux autres produits testés (C et D) montrent une efficacité moindre : pratiquement aucun effet sur la production de céramides, augmentation faible de la production de NMF.

*Tableau 13 : Teneur en NMF et céramides dans les épidermes reconstruits de 1 an incubés en condition normale (contrôle) ou en étuve sèche (atmosphère sèche)*

| | Teneur en NMF ($\mu$g/mg de protéines) | | Teneur en céramides (u.a./mg de protéines) | |
|---|---|---|---|---|
| Contrôle | 16,6±1,8 | | 179,5 | |
| Témoin étuve sèche | 11,7±8,8 | -30% | 138,25 | -23% |
| Produit A | 17,3±3,6 | +48% | 178,25 | +24% |
| Produit B | 18,3±8,1 | +56% | 183,25 | +27% |
| Produit C | 13,9±2 | +19% | 127,3 | -12% |
| Produit D | 14,5±1,7 | +24% | 150,95 | +5% |

### Revendications

1. Procédé d'évaluation de l'efficacité in vitro d'un actif ou d'une formulation sur la prévention des effets de la déshydratation sur la peau d'enfant, **caractérisé en ce que** ledit procédé comprend les étapes suivantes :

   a) mettre en contact ledit actif ou ladite formulation avec un modèle de peau reconstruite, ledit modèle étant obtenu à partir d'un échantillon cutané provenant d'un enfant ;

b) cultiver le modèle de peau reconstruite de l'étape a) dans des conditions de dessiccation ;
c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souches ; et
d) évaluer l'efficacité dudit actif ou de ladite formulation en fonction du niveau de l'étape c).

2. Procédé d'évaluation de l'efficacité in vitro d'un actif ou d'une formulation dans la réduction des effets de la déshydratation sur la peau d'enfant, **caractérisé en ce que** ledit procédé comprend les étapes suivantes :

a) cultiver le modèle de peau reconstruite dans des conditions de dessiccation, ledit modèle étant obtenu à partir d'un échantillon cutané provenant d'un enfant ;
b) mettre en contact ledit actif ou ladite formulation avec le modèle de peau reconstruite de l'étape a) ;
c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et
d) évaluer l'efficacité dudit actif ou de ladite formulation en fonction du niveau de l'étape c).

3. Procédé d'identification d'un actif ou d'une formulation pour la prévention des effets de la déshydratation sur la peau d'enfant, **caractérisé en ce que** ledit procédé comprend les étapes suivantes :

a) mettre en contact un actif ou une formulation candidat avec un modèle de peau reconstruite, ledit modèle étant obtenu à partir d'un échantillon cutané provenant d'un enfant ;
b) cultiver le modèle de peau reconstruite de l'étape a) dans des conditions de dessiccation ;
c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et
d) déterminer si ledit actif ou ladite formulation candidat est une formulation ou un actif pour la prévention des effets de la déshydratation sur la peau d'enfant en fonction du niveau de l'étape c).

4. Procédé d'identification d'un actif ou d'une formulation pour la réduction des effets de la déshydratation sur la peau d'enfant, **caractérisé en ce que** ledit procédé comprend les étapes suivantes :

a) cultiver le modèle de peau reconstruite dans des conditions de dessiccation, ledit modèle étant obtenu à partir d'un échantillon cutané provenant d'un enfant ;
b) mettre en contact un actif ou une formulation candidat avec le modèle de peau de l'étape a) ;
c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et
d) déterminer si ledit actif ou ladite formulation candidat est une formulation ou un actif pour la réduction des effets de la déshydratation sur la peau d'enfant en fonction du niveau de l'étape c).

5. Procédé d'évaluation de la tolérance d'un actif ou d'une formulation sur la peau d'enfant déshydratée, ledit procédé comprenant les étapes suivantes :

a) mettre en contact ledit actif ou ladite formulation avec un modèle de peau reconstruite, ledit modèle étant obtenu à partir d'un échantillon cutané provenant d'un enfant ;
b) cultiver le modèle de peau reconstruite de l'étape a) dans des conditions de dessiccation en présence de l'actif ou de la formulation ;
c) mesurer le niveau d'expression d'une combinaison de marqueurs biologiques dans le modèle de peau de l'étape b), ladite combinaison comprenant au moins un lipide et le NMF et au moins un marqueur de l'inflammation et au moins un marqueur de la barrière et au moins un marqueur préférentiellement exprimé dans les cellules souche; et
d) déterminer si ledit agent actif, l'actif ou de la formulation est bien toléré par la peau d'enfant en fonction du niveau de l'étape c).

**6.** Procédé selon l'une quelconque des revendications 1 à 5, **caractérisé en ce que** le lipide est un céramide, le marqueur de l'inflammation est choisi parmi IL1A, IL8, PTGS2, NGFR, TAC1, TAC1R, TRPV1, TRPV3, MRGPRD, OSMR, PLA2G2F et F2RL1, le marqueur de la fonction barrière est choisi parmi DSG, SCEL, PADI1, CASP14, LOR, TGM1 et CLDN1, et le marqueur préférentiellement exprimé dans les cellules souches est choisi parmi ΔNp63, KRT15, KRT19, BIRC5, et NOTCH1.

**7.** Procédé selon l'une quelconque des revendications 1 à 6, **caractérisé en ce que** l'échantillon cutané provient d'un donneur choisi dans le groupe constitué des nouveau-nés, dont l'âge est compris entre 0 et 1 mois, des nourrissons, dont l'âge est compris entre 1 mois et 2 ans, et des enfants dont l'âge est compris entre 2 ans et 16 ans.

**8.** Procédé selon l'une quelconque des revendications 1 à 7, **caractérisé en ce que** l'échantillon cutané provient d'une peau présentant un phototype I, II, III, IV, V ou VI.

**9.** Procédé selon l'une quelconque des revendications 1 à 8, **caractérisé en ce que** le modèle de peau reconstruite est choisi parmi les cultures de cellules cutanées en suspension, les cultures de cellules cutanées en monocouche, les cultures de cellules cutanées en bicouche, les cultures de peaux reconstruites et les cultures de muqueuses reconstruites.

**10.** Procédé selon la revendication 9, **caractérisé en ce que** les cellules dudit modèle proviennent d'un explant de tissu cutané ou de cellules souches différenciées en cellules cutanées.

**11.** Procédé selon l'une quelconque des revendications 7 à 9, **caractérisé en ce que** ledit modèle comprend au moins des fibroblastes ou des kératinocytes.

**Patentansprüche**

**1.** Verfahren zur Bewertung der In-vitro-Wirksamkeit eines Wirkstoffs oder einer Formulierung zur Vorbeugung der Auswirkungen von Dehydrierung auf die Haut von Kindern, **dadurch gekennzeichnet, dass** das Verfahren die folgenden Schritte umfasst:

a) Inkontaktversetzen des Wirkstoffs oder der Formulierung mit einem rekonstruierten Hautmodell, wobei das Modell aus einer Hautprobe eines Kindes erhalten wird;
b) Kultivieren des rekonstruierten Hautmodells aus Schritt a) unter Trocknungsbedingungen;
c) Messen des Expressionsniveaus einer Kombination biologischer Marker in dem Hautmodell aus Schritt b), wobei die Kombination mindestens ein Lipid und NMF und mindestens einen Entzündungsmarker und mindestens einen Barrieremarker und mindestens einen Marker, der bevorzugt in Stammzellen exprimiert wird, umfasst; und
d) Bewerten der Wirksamkeit des Wirkstoffs oder der Formulierung in Abhängigkeit des Niveaus von Schritt c).

**2.** Verfahren zur Bewertung der In-vitro-Wirksamkeit eines Wirkstoffs oder einer Formulierung zur Verringerung der Auswirkungen von Dehydrierung auf die Haut von Kindern, **dadurch gekennzeichnet, dass** das Verfahren die folgenden Schritte umfasst:

a) Kultivieren des rekonstruierten Hautmodells unter Trocknungsbedingungen, wobei das Modell aus einer Hautprobe eines Kindes erhalten wird;
b) Inkontaktversetzen des Wirkstoffs oder der Formulierung mit dem rekonstruierten Hautmodell aus Schritt a);
c) Messen des Expressionsniveaus einer Kombination biologischer Marker in dem Hautmodell aus Schritt b), wobei die Kombination mindestens ein Lipid und NMF und mindestens einen Entzündungsmarker und mindestens einen Barrieremarker und mindestens einen Marker, der bevorzugt in Stammzellen exprimiert wird, umfasst; und
d) Bewerten der Wirksamkeit des Wirkstoffs oder der Formulierung in Abhängigkeit des Niveaus von Schritt c).

**3.** Verfahren zur Identifizierung eines Wirkstoffs oder einer Formulierung zur Vorbeugung der Auswirkungen von Dehydrierung auf die Haut von Kindern, **dadurch gekennzeichnet, dass** das Verfahren die folgenden Schritte umfasst:

a) Inkontaktversetzen eines Wirkstoff- oder Formulierungskandidaten mit einem rekonstruierten Hautmodell, wobei das Modell aus einer Hautprobe eines Kindes erhalten wird;

b) Kultivieren des rekonstruierten Hautmodells aus Schritt a) unter Trocknungsbedingungen;

c) Messen des Expressionsniveaus einer Kombination biologischer Marker in dem Hautmodell aus Schritt b), wobei die Kombination mindestens ein Lipid und NMF und mindestens einen Entzündungsmarker und mindestens einen Barrieremarker und mindestens einen Marker, der bevorzugt in Stammzellen exprimiert wird, umfasst; und

d) Bestimmen, ob der Wirkstoff- oder der Formulierungskandidat eine Formulierung oder ein Wirkstoff zur Vorbeugung der Auswirkungen von Dehydrierung auf die Haut von Kindern in Abhängigkeit des Niveaus von Schritt c) ist.

4. Verfahren zur Identifizierung eines Wirkstoffs oder einer Formulierung zur Verringerung der Auswirkungen von Dehydrierung auf die Haut von Kindern, **dadurch gekennzeichnet, dass** das Verfahren die folgenden Schritte umfasst:

a) Kultivieren des rekonstruierten Hautmodells unter Trocknungsbedingungen, wobei das Modell aus einer Hautprobe eines Kindes erhalten wird;

b) Inkontaktversetzen eines Wirkstoff- oder eines Formulierungskandidaten mit dem Hautmodell aus Schritt a);

c) Messen des Expressionsniveaus einer Kombination biologischer Marker in dem Hautmodell aus Schritt b), wobei die Kombination mindestens ein Lipid und NMF und mindestens einen Entzündungsmarker und mindestens einen Barrieremarker und mindestens einen Marker, der bevorzugt in Stammzellen exprimiert wird, umfasst; und

d) Bestimmen, ob der Wirkstoff- oder der Formulierungskandidat eine Formulierung oder ein Wirkstoff zur Vorbeugung der Auswirkungen von Dehydrierung auf die Haut von Kindern in Abhängigkeit des Niveaus von Schritt c) ist.

5. Verfahren zur Bewertung der Verträglichkeit eines Wirkstoffs oder einer Formulierung auf dehydrierter Haut von Kindern, wobei das Verfahren die folgenden Schritte umfasst:

a) Inkontaktversetzen des Wirkstoffs oder der Formulierung mit einem rekonstruierten Hautmodell, wobei das Modell aus einer Hautprobe eines Kindes erhalten wird;

b) Kultivieren des rekonstruierten Hautmodells aus Schritt a) unter Trocknungsbedingungen in Gegenwart des Wirkstoffs oder der Formulierung;

c) Messen des Expressionsniveaus einer Kombination biologischer Marker in dem Hautmodell aus Schritt b), wobei die Kombination mindestens ein Lipid und NMF und mindestens einen Entzündungsmarker und mindestens einen Barrieremarker und mindestens einen Marker, der bevorzugt in Stammzellen exprimiert wird, umfasst; und

d) Bestimmen, ob der Wirkstoff oder die Formulierung von der Haut von Kindern in Abhängigkeit des Niveaus von Schritt c) gut vertragen wird.

6. Verfahren nach einem der Ansprüche 1 bis 5, **dadurch gekennzeichnet, dass** das Lipid ein Ceramid ist, der Entzündungsmarker aus IL1A, IL8, PTGS2, NGFR, TAC1, TAC1R, TRPV1, TRPV3, MRGPRD, OSMR, PLA2G2F und F2RL1 ausgewählt ist, der Marker der Barrierefunktion aus DSG, SCEL, PADI1, CASP14, LOR, TGM1 und CLDN1 ausgewählt ist und der in den Stammzellen bevorzugt exprimierte Marker aus $\Delta N\rho 63$, KRT15, KRT19, BIRC5 und NOTCH1 ausgewählt ist.

7. Verfahren nach einem der Ansprüche 1 bis 6, **dadurch gekennzeichnet, dass** die Hautprobe von einem Spender stammt, der aus der Gruppe ausgewählt ist, die aus Neugeborenen, deren Alter zwischen 0 und 1 Monat liegt, Säuglingen, deren Alter zwischen 1 Monat und 2 Jahren liegt, und Kindern, deren Alter zwischen 2 Jahren und 16 Jahren liegt, besteht.

8. Verfahren nach einem der Ansprüche 1 bis 7, **dadurch gekennzeichnet, dass** die Hautprobe von einer Haut stammt, die einen Phototyp I, II, III, IV, V oder VI aufweist.

9. Verfahren nach einem der Ansprüche 1 bis 8, **dadurch gekennzeichnet, dass** das rekonstruierte Hautmodell aus suspendierten Hautzellkulturen, einschichtigen Hautzellkulturen, zweischichtigen Hautzellkulturen, rekonstruierten Hautkulturen und rekonstruierten Schleimhautkulturen ausgewählt ist.

10. Verfahren nach Anspruch 9, **dadurch gekennzeichnet, dass** die Zellen des Modells aus einem Explantat von Hautgewebe oder aus in Hautzellen differenzierten Stammzellen stammen.

**11.** Verfahren nach einem der Ansprüche 7 bis 9, **dadurch gekennzeichnet, dass** das Modell mindestens Fibroblasten oder Keratinozyten umfasst.

**Claims**

**1.** Method for evaluating the in vitro efficacy of an active ingredient or a formulation on preventing the effects of dehydration on children's skin, **characterized in that** said method comprises the following steps:

a) contacting said active ingredient or said formulation with a reconstructed skin model, said model being obtained from a skin sample from a child;
b) growing the reconstructed skin model from step a) under drying conditions;
c) measuring the expression level of a combination of biological markers in the skin model of step b), said combination comprising at least one lipid and NMF and at least one marker of inflammation and at least one marker of the barrier and at least one marker preferentially expressed in stem cells; and
d) evaluating the efficacy of said active or formulation as a function of the level of step c).

**2.** Method for evaluating the in vitro efficacy of an active ingredient or a formulation in reducing the effects of dehydration on children's skin, **characterized in that** said method comprises the following steps:

a) growing the reconstructed skin model under drying conditions, said model being obtained from a skin sample from a child;
b) contacting said active ingredient or formulation with the reconstructed skin model from step a);
c) measuring the expression level of a combination of biological markers in the skin model of step b), said combination comprising at least one lipid and NMF and at least one marker of inflammation and at least one barrier marker and at least one marker preferentially expressed in stem cells; and
d) evaluating the efficacy of said active or formulation as a function of the level of step c).

**3.** Method for identifying an active ingredient or a formulation for preventing the effects of dehydration on children's skin, **characterized in that** said method comprises the following steps:

a) contacting a candidate active or formulation with a reconstructed skin model, said model being obtained from a skin sample from a child;
b) growing the reconstructed skin model from step a) under drying conditions;
c) measuring the expression level of a combination of biological markers in the skin model of step b), said combination comprising at least one lipid and NMF and at least one marker of inflammation and at least one marker of the barrier and at least one marker preferentially expressed in stem cells; and
d) determining whether said candidate active or formulation is a formulation or active for preventing the effects of dehydration on children's skin as a function of the level of step c).

**4.** Method for identifying an active ingredient or a formulation for reducing the effects of dehydration on children's skin, **characterized in that** said method comprises the following steps:

a) growing the reconstructed skin model under drying conditions, said model being obtained from a skin sample from a child;
b) contacting a candidate active or formulation with the skin model from step a);
c) measuring the expression level of a combination of biological markers in the skin model of step b), said combination comprising at least one lipid and NMF and at least one marker of inflammation and at least one marker of the barrier and at least one marker preferentially expressed in stem cells; and
d) determining whether said candidate active or formulation is a formulation or active for reducing the effects of dehydration on children's skin as a function of the level of step c).

**5.** Method for evaluating the tolerance of an active ingredient or a formulation on dehydrated children's skin, said method comprising the following steps:

a) contacting said active ingredient or said formulation with a reconstructed skin model, said model being obtained from a skin sample from a child;

b) growing the reconstructed skin model from step a) under drying conditions in the presence of the active ingredient or formulation;

c) measuring the expression level of a combination of biological markers in the skin model of step b), said combination comprising at least one lipid and NMF and at least one marker of inflammation and at least one marker of the barrier and at least one marker preferentially expressed in stem cells; and

d) determining whether said active agent, active or formulation is well tolerated by the child's skin as a function of the level of step c).

6. Method according to any one of claims 1 to 5, **characterized in that** the lipid is a ceramide, the inflammation marker is selected from IL1A, IL8, PTGS2, NGFR, TAC1, TAC1R, TRPV1, TRPV3, MRGPRD, OSMR, PLA2G2F and F2RL1, the barrier function marker is chosen from DSG, SCEL, PADI1, CASP14, LOR, TGM1 and CLDN1, and the marker preferentially expressed in stem cells is chosen from ΔNp63, KRT15, KRT19, BIRC5, and NOTCH1.

7. Method according to any one of claims 1 to 6, **characterized in that** the skin sample comes from a donor chosen from the group consisting of newborns, whose age is between 0 and 1 month, infants, whose age is between 1 month and 2 years, and children whose age is between 2 years and 16 years.

8. Method according to any one of claims 1 to 7, **characterized in that** the skin sample comes from skin with phototype I, II, III, IV, V or VI.

9. Method according to any one of claims 1 to 8, **characterized in that** the reconstructed skin model is selected from suspension skin cell cultures, monolayer skin cell cultures, bilayer skin cell cultures, reconstructed skin cultures and reconstructed mucosa cultures.

10. Method according to claim 9, **characterized in that** the cells of said model are derived from a skin tissue explant or from stem cells differentiated into skin cells.

11. Method according to any one of claims 7 to 9, **characterized in that** said model comprises at least fibroblasts or keratinocytes.

Figure 1

Figure 2

# EP 3 391 046 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2014009566 A
- WO 2014170495 A
- EP 29678 A
- EP 285471 A
- EP 789074 A
- EP 1451302 B1
- EP 1878790 B1
- EP 1974718 A
- US 20070148771 A
- US 20100099576 A
- WO 02070729 A
- WO 2006063864 A
- WO 2006063865 A
- WO 2007064305 A
- EP 0296078 A1
- WO 01911821 A
- WO 0192322 A
- US 4882127 A
- US 4849077 A
- US 7556922 B
- US 6723513 B
- WO 03066896 A
- WO 2007111924 A
- US 20080020392 A
- WO 2006084132 A
- US 20090186349 A
- US 20090181860 A
- US 20090181385 A
- US 20060275782 A
- EP 1141399 B1
- WO 2004112742 A
- WO 2005105123 A
- WO 2005115421 A
- WO 2008025847 A
- WO 2011073281 A
- WO 2014017049 A
- WO 2014122326 A
- WO 2015044230 A

### Littérature non-brevet citée dans la description

- **HOGAN et al.** *J Allergy*, 2012, vol. 2012, 901940
- **CHIOU et al.** *Skin Pharmacol Physiol*, 2004, vol. 17, 57-66
- **NIKOLOVSKI et al.** *J Invest Dermatol,*, 2008, vol. 128, 1728-1736
- **STAMATAS et al.** *Pediatr Dermatol*, 2010, vol. 27, 125-131
- **TELOFSKI et al.** *Dermatol Res Pract,*, 2012, vol. 2012, 198789
- **FLUHR et al.** *Br J Dermatol*, 2012, vol. 166 (3), 483-90
- **FLUHR et al.** *Br J Dermatol*, 2014
- **YOKOTA et al.** *Experimental Dermatology*, 2014, vol. 23, 27-31
- **DE BENEDETTO et al.** *J Allergy Clin Immunol*, 2011, vol. 127 (3), 773-786
- **YOKOTA et al.** *Exp Dermatol.*, 2014, vol. 23 (1), 27-31
- **FLUHR et al.** *Exp Dermatol*, 2010, vol. 19 (6), 483-492
- **FLUHR et al.** *Br J Dermatol.*, 2014
- **SUNWOO et al.** *J Physiol Anthropol*, 2006, vol. 25, 7-14
- **MOSMAN et al.** *J Immunol Methods*, 1983, vol. 65 (1-2), 55-63
- **GUENOU et al.** *Lancet*, 2009, vol. 374 (9703), 1745-1753
- **NISSAN et al.** *Proc. Natl. Acad. Sci.*, 2011, vol. 108 (36), 14861-14866
- **KRAEHENBUEHL et al.** *Nature Methods*, 2011, vol. 8, 731-736
- **ROSDY et al.** *In Vitro Toxicol.*, 1997, vol. 10 (1), 39-47
- **PONEC et al.** *J Invest Dermatol.*, 1997, vol. 109 (3), 348-355
- **PONEC et al.** *Int J Pharm.*, 2000, vol. 203 (1-2), 211-225
- **SCHMALZ et al.** *Eur J Oral Sci*, 2000, vol. 108 (5), 442-448
- **BLACK et al.** *Tissue Eng*, 2005, vol. 11 (5-6), 723-733
- **DONGARI-BATGTZOGLOU ; KASHLEVA.** *Nat Protoc*, 2006, vol. 1 (4), 2012-2018
- **BECHTOILLE et al.** *Tissue Eng*, 2007, vol. 13 (11), 2667-2679
- **VRANA et al.** *Invest Ophthalmol Vis Sci*, 2008, vol. 49 (12), 5325-5331
- **KINICOGLU et al.** *Biomaterials*, 2009, vol. 30 (32), 6418-6425
- **AUXENFANS et al.** *Eur J Dermatol,*, 2009, vol. 19 (2), 107-113

- **KINICOGLU et al.** *Biomaterials*, 2011, vol. 32 (25), 5756-5764
- **COSTIN et al.** *Altern Lab Anim*, 2011, vol. 39 (4), 317-337
- **AUXENFANS et al.** *J Tissue Eng Regen Med,*, 2012, vol. 6 (7), 512-518
- **LEQUEUX et al.** *Skin Pharmacol Physiol,*, 2012, vol. 25 (1), 47-55
- **MICHEL et al.** *In Vitro Cell. Dev Biol. -Animal*, 1999, vol. 35, 318-326
- **JUNGERSTEND et al.** *Contact Dermitis*, 2008, vol. 58 (5), 255-262
- **DAYAN.** *Cosm & Toil*, 2006, vol. 121 (1), 37-44
- **FARWICK et al.** *Cosm & Toil*, vol. 124 (2), 63-72
- **MASUKAWA et al.** *J Lipid Res.*, 2009, vol. 50 (8), 1708-1719
- **VAHLQUIST.** *Acta Derm Venereol*, 2000, vol. 80, 161
- **SHIMIZU.** *Annu Rev Pharmacol Toxicol.*, 2009, vol. 49, 123-150
- **HOSTE et al.** *J Invest Dermatol.*, 2011, vol. 131 (11), 2233-2241
- **FORTUNE ; MARTIN.** *J Soc Biol*, 2008, vol. 202 (1), 55-65
- **SHENDURE ; JI.** *Nat Biotechnol.*, 2008, vol. 26 (10), 1135-45
- **PIHLAK et al.** *Nat Biotechnol.*, 2008, vol. 26 (6), 676-684
- **FULLER et al.** *Nature Biotechnol.*, 2009, vol. 27 (11), 1013-1023
- **MARDIS.** *Genome Med.*, 2009, vol. 1 (4), 40
- **METZKER.** *Nature Rev. Genet.*, 2010, vol. 11 (1), 31-46
- **SULLIVAN et al.** *Arch Ophthalmol.*, 2002, vol. 120 (12), 1689-99
- **NORDBÄCK et al.** *J. High Resolut. Chromatogr.*, 1999, vol. 22, 483-486
- **TORRES et al.** *J. Chromatogr. A.*, 2005, vol. 1078, 28-34
- **DOWNING et al.** *J Invest Dermatol.*, 1981, vol. 77 (4), 358-360
- **NORDSTROM et al.** *J Invest Dermatol*, 1986, vol. 87 (2), 260-263
- **ROBOSKY et al.** *J Lipid Res.*, 2008, vol. 49 (3), 686-692
- **O'NEILL et al.** *J Chromatogr Sci.*, 1976, vol. 14 (1), 28-36
- **SMEDEN et al.** *J Lipid Res*, 2011, vol. 52 (6), 1211-1221
- **RAINVILLE et al.** *J Proteome Res.*, 2007, vol. 6 (2), 552-558
- **CASTRO-PEREZ et al.** *J Proteome Res.*, 2011, vol. 10 (9), 4281-4290
- **BOUWSTRA et al.** *J Invest Dermatol.*, 1991, vol. 97 (6), 1005-1012
- **VAN SMEDEN et al.** *J Lipid Res.*, 1991, vol. 52 (6), 1211-1221
- **GORCEA et al.** *Int J Pharm.*, 10 November 2011
- **DAEHNHARDT-PFEIFFER et al.** *Skin Pharmacol Physiol.*, 2012, vol. 25 (3), 155-161
- **IWAI et al.** *J Invest Dermatol.*, 26 April 2012
- **CASPERS et al.** *J Invest Dermatol*, 2001, vol. 116 (3), 434-442
- **VYUMVUHORE et al.** *J Biomed Opt.*, 2014, vol. 19 (11), 111603
- **FALCONE et al.** *Skin Pharmacol Physiol,*, 2015, vol. 28, 307-317
- **PIRAUD et al.** *Rapid Commun Mass Spectrom*, 2005, vol. 19 (12), 1587-602
- **PETRITIS et al.** *Journal of Chromatography A*, 1999, vol. 833 (2), 147-155
- **HENRIKSEN et al.** *J Am Soc Mass Spectrom*, 2005, vol. 16 (4), 446-455
- **YANG.** Thesis. University of Bath, 2011, vol. Application of biophysics and bioengineering to the assessment of skin barrier function.
- **EISENBERG et al.** *Trends in Genet*, 2003, vol. 19, 362-365
- **POUMAY et al.** *Arch Dermatol Res*, 2004, vol. 296, 203-11